(19) Europäisches Patentamt
European Patent Office
Office européen des brevets

(11) Publication number: **0 505 108 B1**

(12) **EUROPEAN PATENT SPECIFICATION**

(45) Date of publication of patent specification: **18.10.95**

(51) Int. Cl.⁶: **A61K 7/48**

(21) Application number: **92302171.1**

(22) Date of filing: **13.03.92**

The file contains technical information submitted after the application was filed and not included in this specification

(54) **Compositions for regulating skin wrinkles comprising an Arg-Ser-Arg-Lys based peptide.**

(30) Priority: **21.03.91 US 673381**

(43) Date of publication of application: **23.09.92 Bulletin 92/39**

(45) Publication of the grant of the patent: **18.10.95 Bulletin 95/42**

(84) Designated Contracting States: **DE FR GB IT NL**

(56) References cited:
**EP-A- 0 246 753**
**EP-A- 0 339 905**
**WO-A-90/02800**
**FR-A- 2 654 342**

**PATENT ABSTRACTS OF JAPAN vol. 13, no. 21 (C-560)(3369) 18 January 1989**

**DERWENT JAPANESE PATENTS REPORT volume 92, no 11, Derwent Publications Ltd.,London, GB; AN 92-085852**

(73) Proprietor: **THE PROCTER & GAMBLE COMPANY**
**One Procter & Gamble Plaza**
**Cincinnati,**
**Ohio 45202 (US)**

(72) Inventor: **Fulmer, Andrew Wayne**
**1813 Pheasant Hills Drive**
**Loveland,**
**Ohio 45140 (US)**

(74) Representative: **Brooks, Maxim Courtney et al**
**Procter & Gamble Limited**
**Whitley Road**
**Longbenton**
**Newcastle-upon-Tyne NE12 9TS (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 505 108 B1

Rank Xerox (UK) Business Services
(3.10/3.09/3.3.3)

## Description

The present invention relates to the field of anti-aging of skin. Specifically, the invention relates to novel compositions for effacing and preventing wrinkles in mammalian skin.

Skin is subject to abuse by many extrinsic (environmental) factors as well as intrinsic (aging) factors. A common extrinsic factor is exposure to ultraviolet radiation. Whether extrinsic or intrinsic, the abuse results in wrinkling of the skin as well as loss of youthful color (i.e., the skin "yellows"). To many people, skin wrinkles are a reminder of the disappearance of youth. As a result, the elimination of wrinkles has become a booming business in youth-conscious societies. Treatments range from cosmetic creams and moisturizers to various forms of cosmetic surgery.

The tetrapeptide Arg-Ser-Arg-Lys, a preferred active of the present invention, is a basic fibroblast growth factor (hereinafter b-FGF) derived peptide corresponding to positions 107-110. EP-A-246 753 discloses peptides derived from b-FGF, including the tetrapeptide Arg-Ser-Arg-Lys. The peptides are disclosed as being useful as FGF antagonists, which slow down or decrease cellular activity.

Baird, A., D. Schubert, N. Ling and R. Guillemin, (1988) "Receptor- and Heparin-binding Domains of Basic Fibroblast Growth Factor", Procedures of the National Academy of Sciences, Vol. 85, pp. 2324-2328, discloses b-FGF fragments whose amino acid sequences extend beyond positions 107-110 and act as potent antagonists in b-FGF present assays and as a slight agonist in b-FGF absent assays.

EP-A-339 905 discloses compositions comprising b-FGF or acidic FGF and a retinoid.

It is an object of the present invention to provide a composition for topical application for regulating wrinkles in mammalian skin.

The present invention relates to a composition for topical application for regulating wrinkles in mammalian skin comprising a safe and effective amount of a peptide consisting of the amino acid sequence

$Q^a$ - $(Xaa)_n$ Arg Ser Arg Lys $(Xaa)_n$ - $Q^c$

wherein each amino acid is protected or unprotected, n is independently from 0 to 3, Xaa is independently any amino acid, $Q^a$ is selected from hydrogen and an amino-terminus blocking group, and $Q^c$ is selected from hydrogen and a carboxy-terminus blocking group; and a safe and effective amount of a topical carrier comprising an emollient.

The present invention further relates to a method for effacing skin wrinkles comprising treating the skin with a safe and effective amount of a composition comprising a peptide having the amino acid sequence

$Q^a$ - $(Xaa)_n$ Arg Ser Arg Lys $(Xaa)_n$ - $Q^c$

wherein each amino acid is protected or unprotected, n is independently from 0 to 3, Xaa is independently any amino acid, $Q^a$ is selected from hydrogen and an amino-terminus blocking group, and $Q^c$ is selected from hydrogen and a carboxy-terminus blocking group; and a safe and effective amount of a pharmaceutically acceptable carrier.

As used herein, "topical application" means directly laying on or spreading on outer skin.

As used herein, "cutaneous injection" means introduction of a substance beneath or within the skin by a hypodermic needle.

As used herein, "pharmaceutically-acceptable" means that drugs, medicaments or inert ingredients which the term describes are suitable for use in contact with the tissues of humans and lower animals without undue toxicity, incompatability, instability, irritation, allergic response, and the like, commensurate with a reasonable benefit/risk ratio.

As used herein, "regulating wrinkles" means preventing, retarding, arresting, or reversing the process of wrinkle formation in mammalian skin.

As used herein, "safe and effective amount" means an amount of compound or composition sufficient to significantly induce a positive modification in the condition to be treated, but low enough to avoid serious side effects (at a reasonable benefit/risk ratio).

### Active Agent

As used herein, "anti-wrinkle agent" means a peptide having the amino acid sequence:

$Q^a$ - $(Xaa)_n$ Arg Ser Arg Lys $(Xaa)_n$ - $Q^c$

wherein each n is independently an integer selected from 0, 1, 2 and 3; each amino acid is protected or unprotected, each Xaa is independently any amino acid, $Q^a$ is selected from hydrogen and an amino terminus blocking group; and $Q^c$ is selected from hydrogen and a carboxy-terminus blocking group.

In the above peptide structure, each n is independently preferably preferably 0, 1 or 2, more preferably 0 or 1, most preferably 0.

The amino acids making up the peptide may be any combination of L- and D-configurations. In a preferred embodiment, all the amino acids are in L-configuration.

In the above peptide structure, each amino acid is independently preferably unprotected.

As used herein, "amino acid" refers to any naturally or non-naturally occurring, L- or D- configuration, organic-acid having the structure

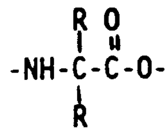

wherein each -R is independently selected from hydrogen and an alkyl having from 1 to 12 carbon atoms. Preferably at least one -R of each amino acid is hydrogen. As used herein, "alkyl" means a carbon-containing chain which may be straight or branched; substituted (mono- or poly-) or unsubstituted; saturated, monounsaturated (i.e., one double or triple bond in the chain), or polyunsaturated (i.e., two or more double bonds in the chain; two or more tripled bonds in the chain; one or more double and one or more triple bonds in the chain). Preferably, the amino acid is naturally occurring. The naturally occurring hydrophobic amino acids are identified by three letter symbols as follows:

| Amino Acid | Three-letter Symbol |
|---|---|
| Alanine | Ala |
| Isoleucine | Ile |
| Leucine | Leu |
| Methionine | Met |
| Phenylalanine | Phe |
| Proline | Pro |
| Hydroxyproline | Hyp |
| Tryptophan | Trp |
| Tyrosine | Tyr |
| Valine | Val |

The naturally occurring hydrophilic amino acids are identified by three letter symbols as follows:

| Amino Acid | Three-letter Symbol |
|---|---|
| Arginine | Arg |
| Asparagine | Asn |
| Aspartic Acid | Asp |
| Asn and/or Asp | Asx |
| Cysteine | Cys |
| Glutamine | Gln |
| Glutamic Acid | Glu |
| Gln and/or Glu | Glx |
| Glycine | Gly |
| Histidine | His |
| Lysine | Lys |
| Hydroxylysine | Hyl |
| Serine | Ser |
| Threonine | Thr |

In a preferred embodiment of the present invention, the peptide comprising the core sequence Arg-Ser-Arg-Lys (SEQ ID NO:1), illustrated as

$$Q^a - \underset{x}{Xaa}\ \underset{y}{Xaa}\ \underset{z}{Xaa}\ \underset{a}{Arg}\ \underset{b}{Ser}\ \underset{c}{Arg}\ \underset{d}{Lys}\ \underset{e}{Xaa}\ \underset{f}{Xaa}\ \underset{g}{Xaa}-Q^c$$

constitutes amino acid positions a through d, respectively, from amino terminus to carboxy terminus. The peptide may have from 0 to 3, preferably 0, amino acids independently bound through amide linkages usual for peptides to the amino terminus and/or the carboxy terminus. Preferably, the z and e position amino acids are hydrophobic, more preferably tyrosine. Preferably, the y and f position amino acids are hydrophilic, more preferably selected from serine and threonine; more preferably still the y position amino acid is threonine and the f position amino acid is serine. Preferably, the x and g position amino acids are hydrophilic; more preferably the x position amino acid is asparagine, and the g position amino acid is serine.

More preferably, the peptide has an amino acid sequence selected from the group consisting of SEQ ID NO: 1, 2, 3, 4, 5, 6, 7, 8, 9, 10, 11, 12, 13, 14, and 15; more preferably still SEQ ID NO: 1, 4, 12 and 15, most preferably SEQ ID NO: 1.

The peptide may also be blocked or unblocked. As used herein, "blocked peptide" means that the amino-terminus and/or the carboxy-terminus of the peptide is bound to a terminus blocking group, i.e., one or both of $Q^a$ or $Q^c$ is not hydrogen. Preferably either $Q^a$ or $Q^c$ is hydrogen; more preferably both $Q^a$ and $Q^c$ are hydrogen.

The amino terminus of the peptide may comprise an amino-terminus blocking group. As used herein, "amino-terminus blocking group" means a fatty acid, free amine, acetamide or a terminal amino protecting group. Examples of terminal amino protecting groups can be found in The Practice of Peptide Synthesis, pp. 9-31 (wherein they are referred to as amine protecting groups) (1984), and are further defined below. Preferably, the fatty acid is saturated or unsaturated, with a carbon chain length of from 2 to 30 carbons, preferably from 8 to 18 carbons, and may be linked to the amino terminus by an amide bond.

The carboxy terminus of the peptide may comprise a carboxy-terminus blocking group. As used herein, "carboxy-terminus blocking group" means a fatty amine, free acid, amide or a terminal carboxyl protecting group. Examples of terminus carboxyl protecting groups can be found in The Practice of Peptide Synthesis, pp. 33-57 (wherein they are referred to as carboxyl protecting groups) (1984), and are further defined below. Preferably, the fatty amine is saturated or unsaturated, with a carbon chain length of from 2 to 30 carbons, preferably from 8 to 18 carbons, and may be linked to the carboxy terminus by an amide bond.

The peptide may also comprise a protecting group on one or more of the amino acid -R moieties in which case, the peptide is referred to as "protected". In the case of tyrosine, the protecting group is preferably selected from tetrahydropyranyl, tertbutyl, trityl, benzyl, carbobenzoxy (CBZ), 4-bromo-carbobenzoxy (4Br-CBZ) and 2,6-dichlorobenzyl. In the case aspartic acid or glutamic acid, the protecting group is preferably selected from benzyl, cyclohexyl, cycloheptal, 2,6-dichlorobenzyl, methyl and ethyl. In the case of cysteine, the protecting group is preferably selected from p-methoxybenzyl, p-methylbenzyl, acetamidomethyl, trityl and benzyl. In the case of threonine or serine, the protecting group is preferably selected from acetyl, benzoyl, tert-butyl, trityl, tetrahydropyranyl, benzyl, 2,6-dichlorobenzyl and CBZ. In the case of arginine, the protecting group is preferably selected from nitro, toluenesulfonyl (Tos), CBZ, acyladamantyloxycarbonyl and t-butyloxycarbonyl (BOC). In the case of lysine, the protecting group is preferably selected from 2-chlorobenzyloxycarbonyl, Tos, CBZ, t-amyloxycarbonyl and BOC. In the case of glutamine or asparagine, the protecting group is preferably xanthyl. In the case of histidine, the protecting group is preferably selected from Tos or dinitrophenyl. A more complete disclosure of such -R protecting groups can be found in The Practice of Peptide Synthesis, pp. 59-85 (1984). As used herein, "unprotected" means the peptide does not comprise any -R protecting groups.

Synthesis of Peptides

It may be preferable to synthesize the peptides of the present invention using recombinant DNA methods. On the other hand, it may be preferable to synthesize the peptides of the present invention using the well known chain elongation techniques such as solid-phase synthesis, as on a Merrifield resin or the like.

To synthesize a peptide using recombinant DNA, one typically synthesizes a double-stranded DNA chain which encodes the desired amino acid sequence. The degeneracy of the genetic code permits a wide variety of codon combinations to be used to form the DNA chain that encodes the product polypeptide. Certain particular codons are more efficient for polypeptide expression in certain types of organisms and

the selection of codons preferably is made according to those codons which are most efficient for expression in the type of organism which is to serve as the host for the recombinant vector. However, any correct set of codons should encode the desired product, even if slightly less efficiently. Codon selection may also depend upon vector construction considerations: for example, it may be necessary to avoid creating a particular restriction site in the DNA chain if, subsequent to insertion of the synthetic DNA chain, the vector is to be manipulated using a restriction enzyme that cleaves at such a site. Also, it is necessary to avoid placing restriction sites in the DNA chain if the host organism which is to be transformed with the recombinant vector containing the DNA chain is known to produce a restriction enzyme that would cleave at such a site within the DNA chain.

In addition to the encoding sequences, the DNA chain that is synthesized may contain additional sequences, depending upon vector construction considerations. Typically, a DNA chain is synthesized with linkers at its ends to facilitate insertion into restriction sites within a cloning vector. The DNA chain may be constructed so as to encode the desired sequence as a portion of a fusion polypeptide and if so, it may contain terminal sequences that encode amino acid residue sequences that serve as proteolytic processing sites, whereby the desired polypeptide may be proteolytically cleaved from the remainder of the fusion polypeptide. The terminal portions of the synthetic DNA chain may also contain appropriate start and stop signals.

To assemble the desired DNA chain, oligonucleotides are constructed by conventional methods such as procedures described in T. Maniatis et al. Cold Spring Harbor Laboratory Manual, Cold Spring Harbor, New York (1982) (hereinafter CSH). Sense and antisense oligonucleotide chains up to about 70 nucleotide residues long are synthesized preferably on automated synthesizers such as the Applied Biosystems in model 380B DNA synthesizer. The oligonucleotide chains are constructed so that the sense and antisense oligonucleotides associate with each other through hydrogen bonding between complementary base pairs and thereby form double stranded chains. These oligonucleotides are then ligated to the vector.

The cloning vector that is to be recombined to incorporate the DNA chain is selected appropriate to its viability and expression in a host organism or cell line, and the manner of insertion of the DNA chain depends upon factors particular to the host. For example, if the DNA chain is to be inserted into a vector for insertion into a prokaryotic cell, such as E. coli, the DNA chain will be inserted 3′ of a promoter sequence, a Shine-Delgarno sequence (or ribosome binding site) that is within a 5′ non-translated portion and an ATG start codon. The ATG start codon is appropriately spaced from the Shine-Delargo sequence, and the encoding sequence is placed in correct reading frame with the ATG start codon. The cloning vector also provides a 3′ non-translated region and a transcription termination site. A translation termination site could be provided by either the synthetic DNA or the cloning vector. For insertion into a eukaryotic cell, such as a yeast cell or a cell line obtained from a higher animal, the encoding oligonucleotide sequence is appropriately spaced from a capping site and in correct reading frame with an ATG start signal. The cloning vector also provides a 3′ non-translated region and a poly adenylation site. A translation termination site could be provided by either the synthetic DNA or the vector.

Derivatives of prokaryotic vectors, such as pBR322, pM89, Col E1, pCRI, RP4 and lambda-phage, are available for inserting a DNA chain of the length necessary to encode peptides of interest with substantial assurance of at least some expression of the encoded polypeptide. Typically, such vectors are constructed or modified to have a unique restriction site(s) appropriately positioned relative to a promoter, such as the lac promoter. The DNA chain may be inserted with appropriate linkers into such a restriction site, with substantial assurance of production of peptide in a prokaryotic cell line transformed with the recombinant vector. To assure the proper reading frame, linkers of various lengths may be provided at the ends of the peptide-encoding sequence. Cassettes, which include sequences, such as the 5′ region of the lac Z gene (including the operator, promoter, transcription start site, Shine Delgarno sequence and translation initiation signal), the regulatory region from the tryptophan gene (trp operator, promoter, ribosome binding site and translation initiator) and a fusion gene containing these two promoters, called the trp-lac or commonly called the Tac promoter, are available into which a synthetic DNA chain may be conveniently inserted.

Similarly, eukaryotic transformation vectors, such as the cloned bovine papilloma virus genome, the cloned genomes of the murine retroviruses, and eukaryotic cassettes, such as the pSV-2 gpt system (described by Mulligan and Berg, Nature, Vol. 277, pp. 108-114, 1979), the Okayama-Berg cloning system (Mol. Cell Biol., Vol. 2, pp. 161-170, 1982) and the expression cloning vector recently described by Genetics Institute (Science, Vol. 228, pp. 810-815, 1985). These provide substantial assurance of at least some expression of the peptide in the transformed eukaryotic cell line.

Another way to produce desired peptides is to produce the polypeptide initially as a segment of a gene-encoded fusion polypeptide. In such case, the DNA chain is constructed so that the expressed polypeptide has enzymatic processing sites flanking the peptide sequence or, more commonly, processing site at one

side of the desired peptide. A peptide-encoding DNA chain may be inserted for example, into the beta-galactosidase gene for insertion into E. coli, in which case, the expressed fusion polypeptide is subsequently cleaved with appropriate proteolytic enzymes to release the peptide from beta-galactosidase peptide sequences.

The peptides can be synthesized by suitable chain elongation or coupling-type methods, such as by exclusively solid-phase techniques, by partial solid-phase techniques, by fragment condensation or by classical solution couplings. The techniques of exclusively solid-phase synthesis are set forth in the textbook "Solid-Phase Peptide Synthesis" Stewart & Young, Pierce Chemical Company, Rockford, Illinois, 1984 and are exemplified by the disclosure of US-A-4,105,603. The fragment condensation method of synthesis is exemplified in US-A-3,972,859. Other available syntheses are exemplified by US-A-3,842,067 and US-A-3,862,925.

Common to coupling-type syntheses is the protection of the labile side chain groups of the various amino acid moieties with suitable protecting groups which will prevent a chemical reaction from occurring at that site until the group is ultimately removed. Usually also common is the protection of an alpha-amino group by an amino-terminus blocking group on an amino acid or a fragment while that entity reacts at the carboxyl group, followed by the selective removal of the amino-terminus blocking group to allow subsequent reaction to take place at that location. Accordingly, it is common that, as a step in the synthesis, an intermediate compound is produced which includes each of the amino acid residues located in its desired sequence in the peptide chain with side-chain protecting groups or terminus blocking group linked to the appropriate residues.

Such an intermediate may have the formula:

$$X^1\text{-Glu}(X^3)\text{-Cys}(X^4)\text{-Tyr}(X^2)\text{-Arg}(X^6)\text{-Ser}(X^5)\text{-Arg}(X^6)\text{-Lys}(X^7)\text{-Asn}(X^8)\text{-His}(X^9)\text{-Asp}(X^3)\text{-}x^{10}$$

In these formulae: $X^1$ is either hydrogen or an amino-terminus blocking group. The amino-terminus blocking groups contemplated by $X^1$ are those known to be useful in the art of step-wise synthesis of polypeptides. Among the classes of amino-terminus blocking groups covered by $X^1$ are (1) acyl-type protecting groups, such as formyl, trifluoroacetyl, phthalyl, toluenesulfonyl (Tos), benzensulfonyl, nitrophenylsulfenyl, tritylsulfenyl, o-nitrophenoxyacetyl, chloroacetyl, acetyl, and y-chlorobutyryl; (2) aromatic urethan-type protecting groups, such as benzyloxycarbonyl (Z) and substituted Z, such as p-chlorobenzyloxycarbonyl, p-nitrobenzyloxycarbonyl, p-bromobenzyloxycarbonyl, p-methoxybenzyloxycarbonyl ; (3) aliphatic urethan protecting groups, such as t-butyloxycarbonyl (BOC), diisopropylmethyloxycarbonyl, isopropyloxycarbonyl, ethoxycarbonyl, allyloxcarbonyl; (4) cycloalkyl urethan-type protecting groups, such as cyclopentyloxcarbonyl, adamantyloxycarbonyl, and cyclohexyloxycarbonyl ; (5) thiourethan-type protecting groups, such as phenylthiocarbonyl; (6) alkyl-type protecting groups, such as triphenylmethyl (trityl), benzyl; (7) trilakylsilane groups, such as trimethylsilane. The preferred amino-terminus blocking group is BOC.

$X^2$ is a protecting group for the phenolic hydroxyl group of Tyr selected from tetrahydropyranyl, tert-butyl, trityl, Bzl, CBZ, 4Br-CBZ and 2,6-dichlorobenzyl . The preferred protecting group is 2,6-dichlorobenzyl. $X^2$ can be hydrogen which means that there is no protecting group on the hydroxyl group.

$X^3$ is hydrogen or an ester-forming protecting group for the carboxyl group of Asp or Glu and is selected from Bzl, cyclohexyl, cycloheptal, 2,6-dichlorobenzyl, methyl and ethyl.

$X^4$ is a protecting group for Cys selected from p-methoxybenzyl(MeOBzl), p-methylbenzyl, acetamidomethyl, trityl and Bzl. The most preferred protecting group is p-methoxybenzyl. $X^4$ can also be hydrogen, meaning that there is no protecting group on the sulfhydryl.

$X^5$ is a protecting group for the hydroxyl group of Thr and Ser and is selected from acetyl, benzoyl, tert-butyl, trityl tetrahydropyranyl, Bzl, 2,6-dichlorobenzyl and CBZ. The preferred protecting group is Bzl. $X^5$ can be hydrogen, which means there is no protecting group on the hydroxyl group.

$X^5$ is a protecting group for the guanido group of Arg selected from nitro, Tos, CBZ, adamantyloxycarbonyl, and BOC, or is hydrogen.

$X^7$ is hydrogen or a protecting group for the side chain amino substituent of Lys. Illustrative of suitable side chain amino protecting groups are 2-chlorobenzyloxycarbonyl(2-Cl-Z), Tos, CBZ, 1-amyloxycarbonyl and BOC.

The selection of a side chain amino protecting group is not critical except that it must be one which is not removed during deprotection of the a-amino groups during the synthesis. Hence, the a-amino protecting

group and the side chain amino protecting group cannot be the same.

$X^8$ is a protecting group for the side chain amido group of Gln and/or Asn and is preferably xanthyl (Xan). Optionally $X^8$ can be hydrogen.

$X^8$ is a protecting group for the imidazole nitrogen of His such as Tos or dinitrophenyl or may be hydrogen.

$X^{10}$ is either hydrogen or a carboxy-terminus blocking group selected from $OCH_2$, esters, amides, hydrazides, -O-$CH_2$- resin support and -NH-resin support.

In the formula for the intermediate, at least one of $X^1$, $X^2$, $X^3$, $X^4$, $X^8$, $X^8$, $X^7$, $X^8$, $X^8$ and $X^{10}$ is an -R protecting group/terminal amino protecting group/terminal carboxyl protecting group.

In selecting a particular side chain protecting group to be used in the synthesis of the peptides, the following rules are followed: (a) the protecting group should be stable to the reagent and under the reaction conditions selected for removing the a-amino protecting group at each step of the synthesis, (b) the protecting group should retain its protecting properties and not be split off under coupling conditions, and (c) the side chain protecting group should be removable, upon the completion of the synthesis containing the desired amino acid sequence, under reaction conditions that will not alter the peptide chain.

The peptides are preferably prepared using solid phase synthesis, such as that described by Merrifield, J. Am. Chem. Soc., Vol. 85, pp. 2149 (1963), although other equivalent chemical syntheses known in the art can also be used as previously mentioned. Solid-phase synthesis is commenced from the C-terminal end of the peptide by coupling a protected a-amino acid to a suitable resin. Such a starting material can be prepared by attaching a-amino-protected Val by an ester linkage to a chloromethylated resin or a hydroxymethyl resin, or by an amide bond to a butylated hydroxy anisole (BHA) resin or MBHA resin. The preparation of the hydroxymethyl resin is described by Bodansky et al., Chem. Ind. (London) Vol. 38, pp. 1597-98 (1966). Chloromethylated resins are commercially available from Bio Rad Laboratories, Richmond, California and from Lab. Systems, Inc. The preparation of such a resin is described by Stewart et al, "Solid Phase Peptide Synthesis" (Freeman & Co., San Francisco 1969), Chapter 1, pp. 1-6. BHA and MBHA resin supports are commercially available and are generally used only when the desired polypeptide being synthesized has an a-carboxamide at the C- terminal.

For example, a peptide of the first family can be prepared by coupling Lys, protected by BOC, to a chloromethylated resin according to the procedure of Monahan and Gilon, Biopolymer, Vol. 12, pp. 2513-19, (1973) when, for example, it is desired to synthesize such a peptide with free carboxy terminus. Following the coupling of BOC-Lys, the a-amino protecting group is removed, as by using trifluoroacetic acid (TFA) in methylene chloride, TFA alone or HCl in dioxane. The deprotection is carried out at a temperature between about 0°C and room temperature. Other standard cleaving reagents and conditions for removal of specific a-amino protecting groups may be used as described in Schroder & Lubke, "The Peptides", pp. 72-75 (Academic Press 1965).

After removal of the a-amino protecting group of Val, the remaining a-amino-and side chain-protected amino acids are coupled stepwise in the desired order to obtain an intermediate compound as defined hereinbefore. As an alternative to adding each amino acid separately in the synthesis, some of them may be coupled to one another prior to their addition to the solid phase reactor. The selection of an appropriate coupling reagent is within the skill of the art; particularly suitable as a coupling reagent is N,N-dicyclohexylcarbodiimide (DCCI).

Activating reagents used in solid phase synthesis of the peptides are well known in the peptide synthesis art. Examples of suitable activating reagents are (1) carbodiimides, such as N,N-diisopropylcarbodiimide, N,N-dicyclohexylcarbodiimide (DCCI); (2) cyanamides such as N,N-dibenzylcyanamide; (3) keteimines; (4) isoxazolium salts, such as N-ethyl-5-phenyl isoxazolium-3-sulfonate; (5) monocyclic nitrogen-containing heterocyclic amides of aromatic character containing one through four nitrogens in the ring, such as imidazolides, pyrazolides, and 1,2,4-triazolides. Specific heterocyclic amides that are useful include N,N-carbonyl diimidazole, N,N-carbonyl-di-1,2,4-triazole; (6) alkoxylated acetylene, such as ethoxyacetylene; (7) reagents which form a mixed anhydride with the carboxyl moiety of the amino acid, such as ethylchloroformate and isobutylchloroformate and (8) reagents which form an active ester with the carboxyl moiety of the amino acid, such as nitrogen-containing heterocyclic compounds having a hydroxy group on one ring nitrogen, e.g., N-hydroxyphthalimide, N-hydroxysuccinimide and 1-hydroxybenzotriazole (HOBT). Other activating reagents and their use in peptide coupling are described by Schroder & Lubke supra, in Chapter III and by Kapoor, J. Phar. Sci., Vol. 59, pp. 1-27 (1970).

Each protected amino acid or amino acid sequence is introduced into the solid phase reactor in about a twofold or more excess, and the coupling may be carried out in a medium of dimethylformamide (DMF)-$CH_2Cl_2$ (1:1) or in DMF or $CH_2Cl_2$ alone. In cases where incomplete coupling occurs, the coupling procedure is repeated before removal of the a-amino protecting group prior to the coupling of the next

amino acid. If performed manually, the success of the coupling reaction at each stage of the synthesis is monitored by the ninhydrin reaction, as described by E. Kaiser et al., Anal. Biochem., Vol. 34, pp. 595 (1970).

After the desired amino acid sequence has been completed, the intermediate peptide is removed from the resin support by treatment with a reagent, such as liquid hydrogen fluoride, which not only cleaves the peptide from the resin but also cleaves all remaining side chain protecting groups $X^2$, $X^3$, $X^4$, $X^5$, $X^5$, $X^7$, $X^5$ and $X^5$ and the a-amino protecting group $X^1$ to obtain the peptide.

As an alternative route, the intermediate peptide may be separated from the resin support by alcoholysis after which the recovered C-terminal alkyl ester is converted to the acid by hydrolysis. Any side chain protecting groups may then be cleaved as previously described or by other known procedures, such as catalytic reduction (e.g., Pd on $BaSO_4$). When using hydrogen fluoride for cleaving, anisole and methylethyl sulfide are included in the reaction vessel for scavenging.

Pharmaceutical Compositions

In addition to the active agent as previously described, the pharmaceutical compositions of the present invention comprise a pharmaceutically-acceptable carrier. The term "pharmaceutically-acceptable carrier", as used herein, means one or more compatible solid or liquid filler diluents which are suitable for administration to a human or lower animal. Pharmaceutically-acceptable carriers must be of sufficiently high purity and sufficiently low toxicity to render them suitable for administration to the human or lower animal being treated. A safe and effective amount of carrier is preferably from 50% to 99.9999%, more preferably from 90% to 99.9% of the composition.

Variations in formulation of these carriers will result in a wide variety of products which fall within the scope of the present invention.

The topical pharmaceutical compositions of the present invention may be made into a wide variety of product types. These include, but are not limited to lotions, creams, beach oils, gels, sticks, sprays, ointments, pastes, mousses and cosmetics. These product types may comprise several types of carrier systems including, but not limited to solutions, emulsions, gels and solids.

The topical pharmaceutical compositions of the present invention formulated as solutions typically include a pharmaceutically-acceptable aqueous or organic solvent. The terms "pharmaceutically-acceptable aqueous solvent" and "pharmaceutically-acceptable organic solvent" refer to a solvent which is capable of having dissolved therein the anti-wrinkle agent, and possesses acceptable safety properties (e.g., irritation and sensitization characteristics). Examples of suitable organic solvents include: propylene glycol, polyethylene glycol (degree of polymerization 200-600), polypropylene glycol (degree of polymerization 425-2025), glycerol, 1,2,4-butanetriol, sorbitol esters, 1,2,6-hexanetriol, ethanol, isopropanol, butanediol, and mixtures thereof. These solutions contain from 0.001% to 20%, more preferably from 0.1% to 10% of the anti-wrinkle agent, and from 80% to 99.999%, more preferably from 90% to 99.9% of an acceptable aqueous or organic solvent.

If the topical pharmaceutical compositions of the present invention are formulated as an aerosol and applied to the skin as a spray-on, a propellant is added to a solution composition. Examples of propellants useful herein include, but are not limited to, the chlorinated, fluorinated and chloro-fluorinated lower molecular weight hydrocarbons. A more complete disclosure of propellants useful herein can be found in Sagarin, Cosmetics Science and Technology, 2nd Edition, Vol. 2, pp. 443-465 (1972).

Topical pharmaceutical compositions of the present invention further comprise from 2% to 50% of a topical pharmaceutically-acceptable emollient. As used herein, "emollients" refer to materials used for the prevention or relief of dryness, as well as for the protection of the skin. A wide variety of suitable emollients are known and may be used herein. Sagarin, Cosmetics, Science and Technology, 2nd Edition, Vol. 1, pp. 32-43 (1972), contains numerous examples of suitable materials. Examples of classes of useful emollients include the following:

1. Hydrocarbon oils and waxes. Examples include mineral oil, petrolatum, paraffin, ceresin, ozokerite, microcrystalline wax, polyethylene, and perhydrosqualene.
2. Silicone oil, such as dimethyl polysiloxanes, methylphenyl polysiloxanes, water-soluble and alcohol-soluble silicone glycol copolymers.
3. Triglyceride esters, for example vegetable and animal fats and-oils. Examples include castor oil, safflower oil, cotton seed oil, corn oil, olive oil, cod liver oil, almond oil, avocado oil, palm oil, sesame oil, and soybean oil.
4. Acetoglyceride esters, such as acetylated monoglycerides.
5. Ethoxylated glycerides, such as ethoxylated glyceryl monostearate.

6. Alkyl esters of fatty acids having 10 to 20 carbon atoms. Methyl, isopropyl, and butyl esters of fatty acids are particularly useful herein. Examples of other useful alkyl esters include hexyl laurate, isohexyl laurate, isohexyl palmitate, isopropyl palmitate, decyl oleate, isodecyl oleate, hexadecyl stearate, decyl stearate, isporopyl isostearate, diisopropyl adipate, dissohexyl adipate, dihexyldecyl adipate, diisopropyl sebacate, lauryl lactate, myristyl lactate, and cetyl lactate.

7. Alkenyl esters of fatty acids having 10 to 20 carbon atoms. Examples include oleyl myristate, oleyl stearate, and oleyl oleate.

8. Fatty acids having 10 to 20 carbon atoms. Suitable examples include pelargonic, lauric, myristic, palmitic, stearic, isostearic, hydroxystearic, oleic, linoleic, ricinoleic, arachidic, behenic, and erucic acids.

9. Fatty alcohols having 10 to 20 carbon atoms. Lauryl, myristyl, cetyl, hexadecyl, stearyl, isostearyl, hydroxystearyl, oleyl, ricinoleyl, behenyl, and erucyl alcohols, as well as 2-octyl dodecanol, are examples of satisfactory fatty alcohols.

10. Fatty alcohol ethers. Ethoxylated fatty alcohols of 10 to 20 carbon atoms include the lauryl, cetyl, stearyl, isostearyl, oelyl, and cholesterol alcohols having attached thereto from 1 to 50 ethylene oxide groups or 1 to 50 propylene oxide groups.

11. Ether-esters such as fatty acid esters of ethoxylated fatty alcohols.

12. Lanolin and derivatives. Lanolin, lanolin oil, lanolin wax, lanolin alcohols, lanolin fatty acids, isopropyl lanolate, ethoxylated lanolin, ethoxylated lanolin alcohols, ethoxolated cholesterol, propoxylated lanolin alcohols, acetylated lanolin, acetylated lanolin alcohols, lanolin alcohols linoleate, lanolin alcohols recinoleate, acetate of lanolin alcohols recinoleate, acetate of lanolin alcohols recinoleate, acetate of ethoxylated alcohols-esters, hydrogenolysis of lanolin, ethoxylated hydrogenated lanolin, ethoxylated sorbitol lanolin, and liquid and semisolid lanolin absorption bases are illustrative of emollients derived from lanolin.

13. Polyhydric alcohols and polyether derivatives. Propylene glycol, dipropylene glycol, polypropylene glycols 2000 and 4000, polyoxyethylene polyoxypropylene glycols, polyoxypropylene polyoxyethylene glycols, glycerol, sorbitol, ethoxylated sorbitol, hydroxypropyl sorbitol, polyethylene glycols 200-6000, methoxy polyethylene glycols 350, 550, 750, 2000 and 5000, poly[ethylene oxide]homopolymers (100,000-5,000,000), polyalkylene glycols and derivatives, hexylene glycol (2-methyl-2,4-pentanediol), 1,3-butylene glycol, 1,2,6-hexanetriol, ethohexadiol USP (2-ethyl-1,3-hexanediol), $C_{15}$-$C_{18}$ vicinal glycol, and polyoxypropylene derivatives of trimethylolpropane are examples of this case of materials.

14. Polyhydric alcohol esters. Ethylene glycol mono- and di-fatty acid esters, diethylene glycol mono- and di-fatty acid esters, polyethylene glycol (200-6000) mono- and di-fatty acid esters, propylene glycol mono- and di-fatty esters, polypropylene glycol 2000 monooleate, polypropylene glycol 2000 monostearate, ethoxylated propylene glycol monostearate, glyceryl mono- and di-fatty acid esters, polyglycerol poly-fatty acid esters, ethoxylated glyceryl monostearate, 1,3-butylene glycol monostearate, 1,3-butylene glycol distearate, polyoxyethylene polyol fatty acid ester, sorbitan fatty acid esters, and polyoxyethylene sorbitan fatty acid esters are satisfactory polyhydric alcohol esters for use herein.

15. Wax esters such as beeswax, spermaceti, myristyl myristate, stearyl stearate.

16. Beeswax derivatives, e.g., polyoxyethylene sorbitol beeswax. These are reaction products of beeswax with ethoxylated sorbitol of varying ethylene oxide content, forming a mixture of ether-esters.

17. Vegetable waxes including carnauba and candelilla waxes.

18. Phospholipids, such as lecithin and derivatives.

19. Sterols. Cholesterol and cholesterol fatty acid esters are examples thereof.

20. Amides such as fatty acid amides, ethoxylated fatty acid amides, solid fatty acid alkanolamides.

Particularly useful emollients which provide skin conditioning are glycerol, hexanetriol, butanetriol, lactic acid and its salts, urea, pyrrolidone carboxylic acid and its salts, amino acids, guanidine, diglycerol and triglycerol. Preferred skin conditioning agents are the propoxylated glycerol derivatives disclosed in US-A-4,976,953. These agents preferably have a formula selected from:

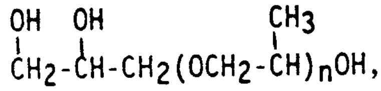

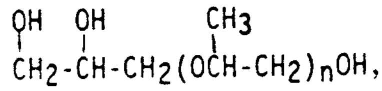

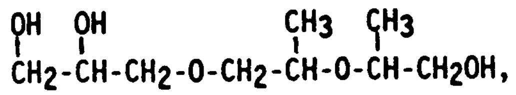

and

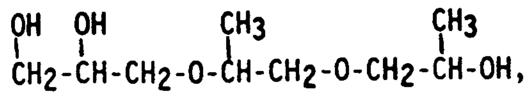

wherein n = 1 or 2, and mixtures thereof. Preferably any of the compositions of the present invention comprises from 1% to 10% by weight of this propoxylated glycerol derivative.

A lotion can be made from a solution carrier system. Lotions typically comprise from 0.001% to 20%, preferably from 0.1% to 10%, of the anti-wrinkle agent; from 1% to 20%, preferably from 5% to 10%, of an emollient; and from 50% to 90%, preferably from 60% to 80%, water.

Another type of product that may be formulated from a solution carrier system is a cream. A cream of the present invention would comprise from 0.001% to 20%, preferably from 0.1% to 10%, of the anti-wrinkle agent; from 5% to 50%, preferably from 10% to 20%, of an emollient, and from 45% to 85%, preferably from 50% to 75%, water.

Yet another type of product that may be formulated from a solution carrier system is an ointment. An ointment may comprise a simple base of animal or vegetable oils or semi-solid hydrocarbons (oleaginous). Ointments may also comprise absorption ointment bases which absorb water to form emulsions. Ointment carriers may also be water soluble. An ointment may also comprise from 2% to 10% of an emollient plus from 0.1% to 2% of a thickening agent. Examples of suitable thickening agents include: cellulose derivatives (e.g., methyl cellulose and hydroxy propylmethyl cellulose), synthetic high molecular weight polymers (e.g., carboxyvinyl polymer and polyvinyl alcohol), plant hydrocolloids (e.g., karaya gum and tragacanth gum), clay thickeners (e.g., colloidal magnesium aliminum silicate and bentonite), and carboxyvinyl polymers (Carbopols® - sold by B. F. Goodrich Company, such polymers are described in detail in US-A-2,789,053. A more complete disclosure of thickening agents useful herein can be found in Segarin, Cosmetics, Science and Technology, 2nd Edition, Vol. 1, pp. 72-73 (1972).

If the carrier is formulated as an emulsion, from 1% to 10%, preferably from 2% to 5%, of the carrier system comprises an emulsifier. Emulsifiers may be nonionic, anionic or cationic. Suitable emulsifiers are disclosed in, for example, U.S. Patent US-A-3,755,560, US-A-4,421,769, and McCutcheon's Detergents and Emulsifiers, North American Edition, pages 317-324 (1986). Preferred emulslfiers are anionic or nonionic, although the other types may also be used.

Lotions and creams can be formulated as emulsions as well as solutions. Typically such lotions comprise from 0.001% to 20%, preferably from 0.01% to 10%, of the anti-wrinkle agent; from 1% to 20%, preferably from 5% to 10%, of an emollient; from 25% to 75%, preferably from 45% to 95%, water; and from 0.1% to 10%, preferably from 0.5% to 5%, of an emulsifier. Such creams would typically comprise from 0.001% to 20%, preferably from 0.01% to 10%, of the anti-wrinkle agent; from 1% to 20%, preferably from 5% to 10%, of an emollient; from 20% to 80%, preferably from 30% to 70%, water; and from 1% to 10%, preferably from 2% to 5%, of an emulsifier.

Single emulsion skin care preparations, such as lotions and creams, of the oil-in-water type and water-in-oil type are well-known in the cosmetic art and are useful in the present invention. Multiphase emulsion compositions, such as the water-in-oil-in-water type, as disclosed in US-A-4,254,105 are also useful in the present invention. In general, such single or multiphase emulsions contain water, emollients and emulsifiers

as essential ingredients.

Triple emulsion carrier systems comprising an oil-in-water-in-silicone fluid emulsion composition as disclosed in US-A-4,960,764 are also useful in the present invention. This triple emulsion carrier system can be combined with from 0.001% to 20%, preferably from 0.01% to 10%, of the anti-wrinkle agent to yield the topical pharmaceutical composition of the present invention.

Another emulsion carrier system useful in the topical pharmaceutical compositions of the present invention is a micro-emulsion carrier system. Such a system preferably comprises from 9% to 15% squalane; from 25% to 40% silicone oil; from 8% to 20% of a fatty alcohol; from 15% to 30% of polyoxyethylene sorbitan mono-fatty acid (commercially available under the trade name Tweens) or other nonionics; and from 7% to 20% water. This carrier system is combined with from 0.01% to 10% of the anti-wrinkle agent.

If the topical pharmaceutical compositions of the present invention are formulated as a gel or a cosmetic stick, a suitable amount of a thickening agent, as disclosed supra, is added to a cream or lotion formulation.

The topical pharmaceutical compositions of the present invention may also be formulated as makeup products such as foundations. Foundations are solution or lotion-based with appropriate amounts of thickeners, pigments and fragrance.

The topical pharmaceutical compositions of the present invention may contain, in addition to the aforementioned components, a wide variety of additional oil-soluble materials and/or water-soluble materials conventionally used in topical compositions, at their art-established levels.

Various water-soluble materials may also be present in the compositions of this invention. These include humectants, such as glycerol, sorbitol, propylene glycol, alkoxylated glucose and hexanetriol, ethyl cellulose, polyvinyl alcohol, carboxymethyl cellulose, vegetable gums and clays such as Veegum® (magnesium aluminum silicate, R. T. Vanderbilt, Inc.); proteins and polypeptides, preservatives such as the methyl, ethyl, propyl and butyl esters of hydroxybenzoic acid (Parabens - Mallinckrodt Chemical Corporation), EDTA, methylisothiazolinone and imidazolidinyl ureas (Germall 115 - Sutton Laboratories); and an alkaline agent such as sodium hydroxide or potassium hydroxide to neutralize, if desired, part of the fatty acids or thickener which may be present. In addition, the topical compositions herein can contain conventional cosmetic adjuvants, such as dyes, opacifiers (e.g., titanium dioxide), pigments and perfumes.

The topical pharmaceutical compositions of the present invention may also include a safe and effective amount of a penetration enhancing agent. A preferred amount of penetration enhancing agent is from 1% to 5% of the composition. Examples of useful penetration enhancers, among others, are disclosed in US-A-4,537,776, US-A-4,552,872, US-A- 4,557,934, US-A-4,130,667, US-A-3,989,816, US-A-4,017,641, and EP-A-0043738.

Other conventional skin care product additives may also be included in the compositions of the present invention. For example, collagen, hyaluronic acid, elastin, hydrolysates, primrose oil, jojoba oil, epidermal growth factor, soybean saponins, mucopolysaccharides, and mixtures thereof may be used.

Various vitamins may also be included in the compositions of the present invention. For example, Vitamin A, and derivatives thereof, Vitamin $B_2$, biotin, pantothenic, Vitamin D, and mixtures thereof may be used.

## Cleaning Compositions

The skin cleaning compositions of the present invention comprise, in addition to the anti-wrinkle agent, a cosmetically-acceptable surfactant. The term "cosmetically-acceptable surfactant" refers to a surfactant which is not only an effective skin cleanser, but also can be used without undue toxicity, irritation, allergic response, and the like. Furthermore, the surfactant must be capable of being commingled with the anti-wrinkle agent in a manner such that there is no interaction which would substantially reduce the efficacy of the composition for regulating wrinkles in mammalian skin.

The skin cleaning compositions of the present invention contain from 0.001% to 20%, preferably from 0.01% to 10%, of the anti-wrinkle agent and from 1% to 90%, preferably from about 5% to about 10%, of a cosmetically-acceptable surfactant.

The physical form of the skin cleansing compositions is not critical. The compositions can be, for example, formulated as toilet bars, liquids, pastes, or mousses. Toilet bars are most preferred since this is the form of cleansing agent most commonly used to wash the skin.

The surfactant component of the compositions of the present invention are selected from anionic, nonionic, zwitterionic, amphoteric and ampholytic surfactants, as well as mixtures of these surfactants. Such surfactants are well-known to those skilled in the detergency art.

The cleaning compositions of the present invention can optionally contain, at their art-established levels, materials which are conventionally used in skin cleansing compositions.

Combination Actives

A. Sunscreens and Sunblocks

Optimum regulation of skin wrinkling resulting from exposure to U.V. light can be obtained by using a combination of the anti-wrinkle agent of the present invention together with sunscreens or sunblocks. Useful sunblocks include, for example, zinc oxide and titanium dioxide.

Photo damage is a predominant cause of skin wrinkling. Thus, for purposes of wrinkle prevention, the combination of the anti-wrinkle agent with a UVA and/or UVB sunscreen would be most desirable. The inclusion of sunscreens in compositions of the present invention at low levels will not significantly reduce the tanning response of the user but will enhance immediate protection against acute UV damage.

A wide variety of conventional sunscreening agents are suitable for use in combination with the anti-wrinkle agent. Segarin, et al., at Chapter VIII, pages 189 et seq., of Cosmetics Science and Technology, disclose numerous suitable agents. Specific suitable sunscreening agents include, for example: p-Aminobenzoic acid, its salts and its derivatives (ethyl, isobutyl, glyceryl esters; p-dimethylaminobenzoic acid); Anthranilates (i.e., o-aminobenzoates; methyl, menthyl, phenyl, benzyl, phenylethyl, linalyl, terpinyl, and cyclohexenyl esters); Salicylates (amyl, phenyl, benzyl, methyl, glyceryl, and dipropyleneglycol esters); Cinnamic acid derivatives (menthyl and benzyl esters, a-phenyl cinnamonitrile; butyl cinnamoyl pyruvate); Dihydroxycinnamic acid derivatives (umbelliferone, methylumbelliferone, methylaceto-umbelliferone); Trihydroxycinnamic acid derivatives (esculetin, methylesculetin, daphnetin, and the glucosides, esculin and daphnin); Hydrocarbons (diphenylbutadiene, stilbene); Dibenzalacetone and benzalacetophenone; Naphtholsulfonates (sodium salts of 2-naphthol-3,6-disulfonic and of 2-naphthol-6,8-disulfonic acids); Dihydroxy-naphthoic acid and its salts; O- and p-Hydroxybiphenyldisulfonates; Coumarin derivatives (7-hydroxy, 7-methyl, 3-phenyl); Diazoles (2-acetyl-3-bromoinda zole, phenyl benzoxazole, methyl naphthoxazole, various aryl benzothiazoles); Quinoline derivatives (8-hydroxyquinoline salts, 2-phenylquinoline); Hydroxy- or methoxy-substituted benzophenones; Uric and vilouric acids; Tannic acid and its derivatives (e.g., hexaethylether); (Butyl carbotol) (6-propyl peperonyl) ether; Hydroquinone; Benzophenones (Oxybenzene, Sulisobenzone, Dioxybenzone, Benzoresorcinol, 2,2',4,4'-Tetrahydroxybenzophenone, 2,2'-Dihydroxy-4,4'-dimethoxybenzophenone, Octabenzone; 4-Isopropyldibenzoylmethane; Butylmethoxydibenxoylmethane; Etocrylene; and 4-isopropyl-di-benxoylmethane.

Of these, 2-ethylhexyl p-methoxycinnamate, 4,4' -t-butyl methoxydibenzoylmethane, 2-hydroxy-4-methoxybenzophenone, octyldimethyl p-aminobenzoic acid, digalloyltrioleate, 2,2-dihydroxy-4-methoxybenzophenone,ethyl 4-[bis(hydroxypropyl)]aminobenzoate, 2-ethylhexyl-2-cyano-e,e-diphenylacrylate, 2-ethylhexylsalicylate, glyceryl p-aminobenzoate, 3,3,5-trimethylcyclohexylsalicylate, 2-ethylhexyl p-dimethylamino-benzoate, 2-phenylbenzimidazole-5-sulfonic acid, 2-(p-dimethylaminophenyl)-5-sulfonicbenzoxazoic acid and mixtures of these compounds, are particularly useful.

Preferred sunscreens useful in the compositions of the present invention are 2-ethylhexyl p-methoxycinnamate, butylmethoxydibenzoylmethane, 2-hydroxy-4-methoxybenzophenone, octyldimethyl p-aminobenzoic acid and mixtures thereof.

A safe and effective amount of sunscreen may be used in the anti-wrinkle agent compositions of the present invention. The sunscreening agent must be compatible with the anti-wrinkle agent. Generally the composition may comprise from 1% to 20%, preferably from 2% to 10%, of a sunscreening agent. Exact amounts will vary depending upon the sunscreen chosen and the desired Sun Protection Factor (SPF).

Also particularly useful in the present invention are sunscreens such as those disclosed in US-A-4,937,380. The sunscreening agents disclosed therein have, in a single molecule, two distinct chromophore moieties which exhibit different ultraviolet radiation absorption spectra. One of the chromophore moieties absorbs predominantly in the UVB radiation range and the other absorbs strongly in the UVA radiation range. Preferred members of this class of sunscreening agents are 4-N,N-(2-ethylhexyl)methylaminobenzoic acid ester of 2,4-dihydroxybenzophenone; N,N-di-(2-ethylhexyl)-4-aminobenzoic acid ester with 4-hydroxydibenzoylmethane; 4-N,N-(2-ethylhexyl) methylaminobenzoic acid ester with 4-hydroxydibenzoylmethane; 4-N,N-(2-ethylhexyl)methylaminobenzoic acid ester of 2-hydroxy-4-(2-hydroxyethoxy)benzophenone; 4-N,N-(2-ethylhexyl) methylaminobenzoic acid ester of 4 -(2-hydroxyethoxy)dibenzoylmethane; N-N-di-(2-ethylhexyl)-4-aminobenzoic acid ester of 2-hydroxy-4-(2-hydroxyethoxy)benzophenone; and N,N-di-(2-ethylhexyl)-4-aminobenzoic acid ester of 4 -(2-hydroxyethoxy)dibenzoylmethane and mixtures thereof.

An agent may also be added to any of the compositions of the present invention to improve the skin substantivity of those compositions, particularly to enhance their resistance to being washed off by water, or rubbed off. A preferred agent which will provide this benefit is a copolymer of ethylene and acrylic acid. Compositions comprising this copolymer are disclosed in US-A-4,663,157.

B. Anti-Inflammatory Agents

In a preferred anti-wrinkle composition of the present invention, an anti-inflammatory agent is included as an active along with the anti-wrinkle agent. The inclusion of an anti-inflammatory agent enhances the wrinkling regulating benefits of the compositions. The anti-inflammatory agent protects strongly in the UVA radiation range (though it also provides some UVB protection as well) thereby preventing further wrinkle formation caused by UV radiation, while the anti-wrinkle agent regulates existing wrinkles. Thus the combination provides broad protection. The topical use of anti-inflammatory agents reduces photo-aging of the skin resulting from chronic exposure to UV radiation. (See US-A-4,847,071 and US-A-4,847,069.)

A safe and effective amount of an anti-inflammatory agent may be added to the compositions of the present invention, preferably from 0.1% to 10%, more preferably from 0.5% to 5%, of the composition. The exact amount of anti-inflammatory agent to be used in the compositions will depend on the particular anti-inflammatory agent utilized since such agents vary widely in potency.

Steroidal anti-inflammatory agents, including but not limited to, corticosteroids such as hydrocortisone, hydroxyltriamcinolone, alpha-methyl dexamethasone, dexamethasone-phosphate, beclomethasone dipropionate, clobetasol valerate, desonide, desoxymethasone, desoxycorticosterone acetate, dexamethasone, dichlorisone, diflorasone diacetate, diflucortolone valerate, fluadrenolone, fluclorolone acetonide, fludrocortisone, flumethasone pivalate, fluosinolone acetonide, fluocinonide, flucortine butylester, fluocortolone, fluprednidene (fluprednylidene) acetate, flurandrenolone, halcinonide, hydrocortisone acetate, hydrocortisone butyrate, methylprednisolone, triamcinolone acetonide, cortisone, cortodoxone, flucetonide, fludrocortisone, difluorosone diacetate, fluradrenolone acetonide, medrysone, amcinafel, amcinafide, betamethasone and the balance of its esters, chloroprednisone, chlorprednisone acetate, clocortelone, clescinolone, dichlorisone, difluprednate, flucloronide, flunisolide, fluoromethalone, fluperolone, fluprednisolone, hydrocortisone valerate, hydrocortisone cyclopentylpropionate, hydrocortamate, meprednisone, paramethasone, prednisolone, prednisone, beclomethasone dipropionate, triamcinolone, and mixtures thereof may be used. The preferred steroidal anti-inflammatory for use in the present invention is hydrocortisone.

A second class of anti-inflammatory agents which is useful in the compositions of the present invention includes the nonsteroidal anti-inflammatory agents. The variety of compounds encompassed by this group are well-known to those skilled in the art. For detailed disclosure of the chemical structure, synthesis, side effects, etc., of non-steroidal anti-inflammatory agents, reference may be had to standard texts, including Anti-inflammatory and Anti-Rheumatic Drugs, K. D. Rainsford, Vol. I-III, CRC Press, Boca Raton, (1985), and Anti-inflammatory Agents, Chemistry and Pharmacology, 1, R. A. Scherrer , et al., Academic Press, New York (1974).

Specific non-steroidal anti-inflammatory agents useful in the composition of the present invention include, but are not limited to:

1) the oxicams, such as piroxicam, isoxicam, tenoxicam, sudoxicam, and CP-14,304;
2) the salicylates, such as aspirin, disalcid, benorylate, trilisate, safapryn, solprin, diflunisal, and fendosal;
3) the acetic acid derivatives, such as diclofenac, fenclofenac, indomethacin, sulindac, tolmetin, isoxepac, furofenac, tiopinac, zidometacin, acematacin, fentiazac, zomepiract, clidanac, oxepinac, and felbinac;
4) the fenamates, such as mefenamic, meclofenamic, flufenamic, niflumic, and tolfenamic acids;
5) the propionic acid derivatives, such as ibuprofen, naproxen, benoxaprofen, flurbiprofen, ketoprofen, fenoprofen, fenbufen, indoprofen, pirprofen, carprofen, oxaprozin, pranoprofen, miroprofen, tioxaprofen, suprofen, alminoprofen, and tiaprofenic; and
6) the pyrazoles, such as phenybutazone, oxyphenbutazone, feprazone, azapropazone, and trimethazone.

Mixtures of these non-steroidal anti-inflammatory agents may also be employed, as well as the pharmaceutically-acceptable salts and esters of these agents. For example, etofenamate, a flufenamic acid derivative, is particularly useful for topical application. Of the nonsteroidal anti-inflammatory agents, ibuprofen, naproxen, flufenamic acid, mefenamic acid, meclofenamic acid, piroxicam and felbinac are preferred; ibuprofen, naproxen, and flufenamic acid are most preferred.

Another class of anti-inflammatory agents which are useful in the present invention are the anti-inflammatory agents disclosed in US-A-4,708,966. This patent discloses a class of nonsteroidal anti-inflammatory compounds which comprise specifically substituted phenyl compounds, especially substituted 2,6-di- tert-butyl phenol derivatives. For example, compounds selected from 4 -(4'-pentyn-3'-one)-2,6-di-t-butylphenol; 4-(5'-hexynoyl)2,6 -di-t-butylphenol; 4-((S)-(-)-3'-methyl-5'-hexynoyl)-2,6-di-t-butylphenol; 4-((R)-(+)-3'-methyl-5'-hexynoyl)-2,6-di-t-butylphenol; and 4-(3' ,3'-dimethoxypropionyl)-2,6-di-t-butylphenol are useful in the present invention.

Yet another class of anti-inflammatory agents which are useful in the present invention are those disclosed in US-A-4,912,248. This patent discloses compounds and diastereomeric mixtures of specific 2-naphthyl- containing ester compounds, especially naproxen ester and naproxol ester compounds, having two or more chiral centers. For example, compounds selected from (S)-naproxen-(S)-2-butyl ester, (S)-naproxen-(R)-2-butylester, (S)-naproxol-(R)-2-methyl butyrate, (S)-naproxol-(S)-2-methyl butyrate, diasteromeric mixtures of (S)-naproxen-(S)-2-butyl ester and (S)-naproxen- (R)-2-butyl ester, and diasteromeric mixtures of (S)-naproxol- (R)-2-methyl butyrate and (S)-naproxol-(S)-2-methyl butyrate are useful in the present invention.

Finally, so-called "natural" anti-inflammatory agents are useful in the present invention. For example, candelilla wax, alpha bisabolol, aloe vera, Manjistha (extracted from plants in the genus Rubia, particularly Rubia Cordifolia), and Guggal (extracted from plants in the genus Commiphora, particularly Commiphora Mukul), may be used.

Another preferred composition of the present invention comprises an anti-wrinkle agent, a sunscreen, and an anti-inflammatory agent together for wrinkle regulation in the amounts disclosed for each individually hereinabove.

C. Anti-Oxidants/Radical Scavengers

In a preferred anti-wrinkle composition of the present invention, an anti-oxidant/radical scavenger is included as an active along with the anti-wrinkle agent. The inclusion of an anti-oxidant/radical scavenger increases the wrinkle regulating benefits of the composition.

A safe and effective amount of an anti-oxidant/radical scavenger may be added to the compositions of the present invention, preferably from 0.1% to 10%, more preferably from 1% to 5%, of the composition.

Anti-oxidants/radical scavengers such as ascorbic acid (vitamin C) and its salts, tocopherol (vitamin E), tocopherol sorbate, other esters of tocopherol, butylated hydroxy benzoic acids and their salts, 6-hydroxy-2,5,7,8-tetramethylchroman-2-carboxylic acid (commercially available under the tradename TroloxR), gallic acid and its alkyl esters, especially propyl gallate, uric acid and its salts and alkyl esters, sorbic acid and its salts, the ascorbyl esters of fatty acids, amines (e.g., N,N-diethylhydroxylamine, amino-guanidine), sulfhydryl compounds (e.g., glutathione), and dihydroxy fumaric acid and its salts may be used.

In a preferred anti-wrinkle composition of the present invention, compositions comprise one, any two, or all three of a sunscreening agent, anti-inflammatory agent, and/or an anti-oxidant/radical scavenging agent included as actives along with the anti-wrinkle agent. The inclusion of two or all three of these agents with the anti-wrinkle agent increases the wrinkle regulating benefits of the composition.

D. Chelators

In a preferred anti-wrinkle composition of the present invention, a chelating agent is included as an active along with the anti-wrinkle agent. As used herein, "chelating agent" means an active agent capable of removing a metal ion from a system by forming a complex so that the metal ion cannot readily participate in or catalyze chemical reactions. The inclusion of a chelating agent increases the wrinkle regulating benefits of the composition.

A safe and effective amount of a chelating agent may be added to the compositions of the present invention, preferably from 0.1% to 10%, more preferably from 1% to 5%, of the composition. Preferred chelators useful in compositions of the present invention are furildioxime and derivatives thereof, more preferably amphi-2-furildioxime.

In a preferred anti-wrinkle composition of the present invention, compositions comprise one, any two, any three, or all four of a sunscreening agent, anti-inflammatory agent, anti-oxidant/radical scavenging agent, and/or chelating agent included as actives along with the anti-wrinkle agent. The inclusion of two, three, or all four of these agents with the anti-wrinkle agent increases the wrinkle regulating benefits of the composition.

E. Retinoids

In a preferred anti-wrinkle composition of the present invention, a retinoid, preferably retinoic acid, is included as an active along with the anti-wrinkle agent. The inclusion of a retinoid increases the wrinkle

regulating benefits of the composition. A safe and effective amount of a retinoid may be added to the compositions of the present invention, preferably from 0.001% to 2%, more preferably from 0.01% to 1% of the composition. As used herein, "retinoid" includes all natural and/or synthetic analogs of Vitamin A or retinol-like compounds which possess the biological activity of Vitamin A in the skin as well as the geometric isomers and stereoisomers of these compounds, such as all-trans retinoic acid and 13-cis-retinoic acid.

In a preferred anti-wrinkle composition of the present invention, compositions comprise one, any two, any three, any four, and/or all five of a sunscreening agent, anti-inflammatory agent, anti-oxidant/radical scavenging agent, chelating agent, and/or a retinoid included as actives along with the anti-wrinkle agent. The inclusion of two, three, four, or all five of these agents with the anti-wrinkle agent increases the wrinkle regulating benefits of the composition.

E. Benzofuran Derivatives

In a preferred anti-wrinkle composition of the present invention, a benzofuran derivative, preferably amiodarone, is included as an active along with the anti-wrinkle agent. The inclusion of a benzofuran derivative increases the wrinkle regulating benefits of the composition.

A safe and effective amount of a benzofuran derivative may be added to the compositions of the present invention, preferably from .01% to 20%, more preferably from 0.1% to 10%, of the composition.

In a preferred anti-wrinkle composition of the present invention, compositions comprise one, any two, any three, any four, any five, and or all six of a sunscreening agent, anti-inflammatory agent, anti-oxidant/radical scavenging agent, chelating agent, retinoid, and/or benzofuran derivative included as actives along with the anti-wrinkle agent. The inclusion of two, three, four, five or all six of these agents with the anti-wrinkle agent increases the wrinkle regulating benefits of the composition.

## Methods for Regulating Wrinkles in Mammalian Skin

A method for regulating wrinkles in mammalian skin comprises treating the skin with a safe and effective amount of the anti-wrinkle agent. The amount of anti-wrinkle agent and frequency of treatment will vary widely depending upon the level of wrinkling already in existence in the subject, the rate of further wrinkle formation, and the level of regulation desired.

A preferred method of treating the skin is via cutaneous injection of a safe and effective amount of the anti-wrinkle agent to regulate wrinkles in mammalian skin. The carrier for injectable administration of the anti-wrinkle agent would preferably comprise water or a saline solution. The amount of anti-wrinkle agent and the frequency of cutaneous injection can vary widely, depending on personal needs. As an example of treatment by cutaneous injection, it is suggested that a composition suitable for cutaneous injection comprising the anti-wrinkle agent be cutaneously injected from once per day to once every six months, preferably from three times per week to once per month, more preferably from once per week to twice per month. The composition for cutaneous injection will contain from about 0.0001% to about 10%, preferably from about 0.001% to about 5%, more preferably from about 0.01% to about 1% of the anti-wrinkle agent. The period of injections would be over a period of from about one month to about ten years, preferably from about three months to about two years, more preferably from about six months to about one year, thereby resulting in regulation of wrinkles in mammalian skin.

A more preferred method of treating the skin is via topical application of a safe and effective amount of the anti-wrinkle agent to regulate wrinkles in mammalian skin. The amount of anti-wrinkle agent and frequency of topical application to the skin can vary widely, depending upon personal needs, but it is suggested as an example that topical application range from about once per week to about 10 times daily, preferably from about twice per week to about 4 times daily, more preferably from about 3 times a week to about twice daily, most preferably about once per day. The composition for topical application will comprise from about 0.001% to about 20%, preferably from about 0.01% to about 10%, more preferably from about 0.1% to about 5% of the anti-wrinkle agent. The period of topical application would preferably be over a period of from about one month to about ten years, more preferably from about three months to about two years, more preferably still from about six months to about one year, thereby resulting in regulation of wrinkles in mammalian skin.

A preferred method for regulating wrinkles in mammalian skin involves applying both a safe and effective amount of the anti-wrinkle agent and a safe and effective amount of one or more of a sunscreening agent, an anti-inflammatory agent, an anti-oxidant/radical scavenging agent, a chelating agent, a retinoid and/or a benzofuran derivative to the skin simultaneously. As used herein, "simultaneous application" or

"simultaneously" means applying the agents to the skin at the same situs on the body at about the same time. Though this can be accomplished by applying the agents separately to the skin, preferably a composition comprising all the desired agents commingled is applied to the skin. The amount of sunscreening agent applied is generally from about 0.02 mg to about 1.0 mg per $cm^2$ skin. The amount of anti-inflammatory agent applied is generally from about 0.005 mg to about 0.5 mg, preferably from about 0.01 mg to about 0.1 mg per $cm^2$ skin. The amount of anti-oxidant/radical scavenging agent generally applied is from about 0.001 mg to about 1.0 mg, preferably from about 0.05 mg to about 0.5 mg per $cm^2$ skin. The amount of chelating agent generally applied is from about .001 mg to about 1.0 mg, preferably from about 0.01 mg to about 0.5 mg, more preferably from about 0.05 mg to about 0.1 mg per $cm^2$ skin. The amount of retinoid applied is generally from about 0.00001 mg to about 0.02 mg per $cm^2$ skin, preferably from about 0.001 mg to about 0.01 mg per $cm^2$ skin. The amount of benzofuran derivative applied is generally from about 0.001 mg to about 1.0 mg/$cm^2$ skin per application, preferably from about 0.01 to about 0.5 mg/$cm^2$ skin per application. The amount of anti-wrinkle agent applied is generally from about 0.001 mg to about 2 mg per $cm^2$ skin per application, preferably from about 0.01 mg to about 1 mg per $cm^2$ skin per application.

The following examples further describe and demonstrate the preferred embodiments within the scope of the present invention. These preparations may be formulated over a range of pH's, preferably pH 3-10.

EXAMPLE I

An oil-in-water emulsion is prepared by combining the following components utilizing conventional mixing techniques.

| Component | Percent by Weight of Composition |
|---|---|
| Deionized water | Quantum sufficit |
| Glycerin | 3 |
| Methyl paraben | 0.2 |
| Arg Ser Arg Lys (SEQ ID NO:1) (L-form, unblocked and unprotected) | 0.1 |
| Steareth 20 (Brij 78®) | 1 |
| Glyceryl monostearate and PEG 100 (Arlacel 165®) | 0.5 |
| Carbopol 940 (B.F. Goodrich, Cleveland, OH) | 0.2 |
| 99% triethanolamine | 0.2 |
| Cetyl alcohol | 1.0 |
| Stearyl alcohol | 1.0 |
| Propyl paraben | 0.1 |
| Disiopropyldimerate | 2.0 |
| $C_{12}$-$C_{15}$ alcohol benzoate | 6.0 |
| Imidazolidinol urea | 0.3 |

This composition Is useful for topical application to regulate skin wrinkles. An amount of the composition sufficient to deposit about 0.01 mg/$cm^2$ of the anti-wrinkle agent to the skin is used. The composition is applied once per day for the subject's lifetime.

EXAMPLE II

A clear gel is prepared by combining the following components utilizing conventional mixing techniques.

| Component | Percent by Weight of Composition |
|---|---|
| Deionized water | Quantum sufficit |
| Carbopol 980 (B.F. Goodrich, Cleveland, OH) | 0.5 |
| Disodium EDTA | 0.02 |
| n-$C_{20}H_{41}$- Arg Ser Arg Lys-n- $C_{18}H_{39}$ (L-form, unblocked and unprotected | 0.5 |
| 995 triethanolamine | 0.5 |
| Propylene glycol | 3.0 |
| Methyl paraben | 0.2 |

This composition is useful for topical application to regulate skin wrinkles. An amount of the composition sufficient to deposit about 0.01 mg/cm$^2$ of anti-wrinkle agent to the skin is used. The composition is applied three times per day over a six-month period.

EXAMPLE III

An oil-in-water polymer emulsion is prepared by combining the following components utilizing conventional mixing techniques.

| Component | Percent by Weight of Composition |
|---|---|
| Deionized water | Quantum sufficit |
| Carbopol 954 (B.F. Goodrich, Cleveland, OH) | 0.2 |
| Pemulen TR-2 (B.F. Goodrich, Cleveland, OH) | 0.15 |
| Glycerin | 3.0 |
| Tos-Arg Ser Arg Lys Tyr $B_z$1 (serine in D-form) | 0.75 |
| 99% triethanolamine | 0.35 |
| Cetyl palmitate | 2.0 |
| Stearaxy trimethyl silane and stearyl alcohol | 1.0 |
| Squalane | 6.0 |
| Propyl paraben | 0.1 |
| Methyl paraben | 0.2 |
| Imidazolidinol urea | 0.3 |

This composition is useful for topical application to regulate skin wrinkles. An amount of the composition sufficient to deposit 0.1 mg/cm$^2$ anti-wrinkle agent to the skin is used. The composition is applied once per week over a one-year period.

EXAMPLE IV

An oil-in-water microemulsion is prepared by combining the following components utilizing conventional mixing techniques.

| Component | Percent by Weight of Composition |
|---|---|
| Deionized water | Quantum sufficit |
| Tyr Arg Ser Arg Lys (SEQ ID NO:2) (L-form, unblocked and unprotected) | 1.0 |
| PEG4 sorbitan monolaurate | 22.5 |
| PEG5 sorbitant monooleate | 2.5 |
| Cetearyl octanoate | 25.0 |
| DMDM hydantoin and 3-iodo-2-propynyl butyl carbamate (glydant plus) | 0.2 |

This composition is useful for topical application to regulate skin wrinkles. An amount of the composition sufficient to deposit 0.4 mg/cm$^2$ anti-wrinkle agent to the skin is used. The composition is applied three times per week over a five-year period.

Compositions of the present invention may also be used in conjunction with various surgical procedures for wrinkle removal such as chemical peel, dermabrasion, laser abrasion and collagen injections.

## Claims

**1.** A composition for topical application for regulating wrinkles in mammalian skin characterized in that it comprises

a. a peptide, preferably from 0.001% to 20%, consisting of the amino acid sequence

$Q^a$ - $(Xaa)_n$ Arg Ser Arg Lys $(Xaa)_n$ - $Q^c$

wherein each n is independently an integer selected from 0, 1, 2 and 3, preferably 0;

each Xaa is independently any amino acid, preferably a naturally occurring amino acid, preferably in L-configuration;

each amino acid is protected or unprotected, preferably unprotected, wherein

any protecting group for Tyr is selected from tetrahydropyranyl, tert-butyl, trityl, Bzl, CBZ, 4Br-CBZ and 2,6-dichlorobenzyl;

any protecting group for Asp or Glu is selected from Bzl, cyclohexyl, cycloheptal, 2,6-dichlorobenzyl, methyl and ethyl;

any protecting group for Cys is selected from MeOBzl, p-methylbenzyl, acetamidomethyl, trityl and Bzl;

any protecting group for Thr or Ser is selected from acetyl, benzoyl, tert-butyl, trityl tetrahydropyranyl, Bzl, 2,6-dichlorobenzyl and CBZ;

any protecting group for Arg is selected from nitro, Tos, CBZ, adamantyloxycarbonyl and BOC;

any protecting group for Lys is selected from 2-Cl-Z, Tos, CBZ, 1-amyloxycarbonyl and BOC;

any protecting group for Gln or Asn is Xan;

any protecting group for His is selected from Tos and dinitrophenyl;

$Q^a$ is selected from hydrogen and an amino-terminus blocking group, preferably hydrogen, wherein

any amino-terminus blocking group is selected from a fatty acid from 2 to 30 carbons, free amine, acetamide and a terminal amino protecting group wherein the terminal amino protecting group is selected from acyl-type blocking groups, aromatic urethan-type blocking groups, aliphatic urethan-type blocking groups, cycloalkyl urethan-type blocking groups, cycloalkyl urethan-type blocking groups, thiourethan-type blocking groups, alkyl-type blocking groups, and trialkylsilane blocking groups; preferably a fatty acid;

$Q^c$ is selected from hydrogen and a carboxy-terminus blocking group, preferably hydrogen, wherein

any carboxy-terminus blocking group is selected from a fatty amine from 2 to 30 carbons, free acid, amide and a terminal carboxyl protecting group wherein the terminal carboxyl protecting group is selected from $-OCH_2$, esters, amides, hydrozides, $-O-CH_2-$ resin support, and -NH-resin support; preferably a fatty amine; and

b. a safe and effective amount of a topical carrier comprising an emollient.

**2.** The composition according to Claim 1 wherein the peptide is Arg Ser Arg Lys (SEQ ID NO:1).

**3.** The composition of any one of the preceeding claims which additionally comprises a safe and effective amount of a sunscreening agent.

**4.** The composition of any one of the preceeding claims which additionally comprises a safe and effective amount of a retinoid.

**5.** The composition of any one of the preceeding claims which additionally comprises a safe and effective amount of a chelator.

6. The composition of any one of the preceeding claims which additionally comprises a safe and effective amount of an anti-inflammatory agent.

7. The composition of any one of Claims 1-2 which additionally comprises a safe and effective amount of a sunscreening agent and a safe and effective amount of a retinoid.

8. The composition of any one of Claims 1-2 which additionally comprises a safe and effective amount of a sunscreening agent and a safe and effective amount of a chelator.

9. The composition of any of Claims 1-2 which additionally comprises a safe and effective amount of a sunscreening agent and a safe and effective amount of an anti-inflammatory agent.

10. The composition according to any one of the preceeding claims for topical use in regulating skin wrinkles in mammalian skin.

11. Use of

a. a peptide, preferably from 0.001% to 20% consisting of the amino acid sequence

$Q^a$ - $(Xaa)_n$ Arg Ser Arg Lys $(Xaa)_n$ - $Q^c$

wherein each n is independently an integer selected from 0, 1, 2 and 3, preferably 0;

each Xaa is independently any amino acid, preferably a naturally occurring amino acid, preferably in L-configuration;

each amino acid is protected or unprotected, preferably unprotected, wherein

any protecting group for Tyr is selected from tetrahydropyranyl, tert-butyl, trityl, Bzl, CBZ, 4Br-CBZ and 2,6-dichlorobenzyl;

any protecting group for Asp or Glu is selected from Bzl, cyclohexyl, cycloheptal, 2,6-dichlorobenzyl, methyl and ethyl;

any protecting group for Cys is selected from MeOBzl, p-methylbenzyl, acetamidomethyl, trityl and Bzl;

any protecting group for Thr or Ser is selected from acetyl, benzoyl, tert-butyl, trityl tetrahydropyranyl, Bzl, 2,6-dichlorobenzyl and CBZ;

any protecting group for Arg is selected from nitro, Tos, CBZ, adamantyloxycarbonyl and BOC;

any protecting group for Lys is selected from 2-Cl-Z, Tos, CBZ, 1-amyloxycarbonyl and BOC;

any protecting group for Gln or Asn is Xan;

any protecting group for His is selected from Tos and dinitrophenyl;

$Q^a$ is selected from hydrogen and an amino-terminus blocking group, preferably hydrogen, wherein

any amino-terminus blocking group is selected from a fatty acid from 2 to 30 carbons, free amine, acetamide and a terminal amino protecting group wherein the terminal amino protecting group is selected from acyl-type blocking groups, aromatic urethan-type blocking groups, aliphatic urethan-type blocking groups, cycloalkyl urethan-type blocking groups, cycloalkyl urethan-type blocking groups, thiourethan-type blocking groups, alkyl-type blocking groups, and trialkylsilane blocking groups; preferably a fatty acid;

$Q^c$ is selected from hydrogen and a carboxy-terminus blocking group, preferably hydrogen, wherein

any carboxy-terminus blocking group is selected from a fatty amine from 2 to 30 carbons, free acid, amide and a terminal carboxyl protecting group wherein the terminal carboxyl protecting group is selected from $-OCH_2$, esters, amides, hydrozides, $-O-CH_2-$ resin support, and -NH-resin support; preferably a fatty amine; and

b. a safe and effective amount of a topical carrier comprising an emollient. in the manufacture of a composition for topical application for regulating wrinkles in mammalian skin

## Patentansprüche

1. Zusammensetzung für die äußerliche Anwendung zur Regulierung von Falten in der Haut von Säugern, **dadurch gekennzeichnet**, daß sie

a. ein Peptid, vorzugsweise 0,001% bis 20%, bestehend aus der Aminosäuresequenz

$Q^a$ - $(Xaa)_n$ Arg Ser Arg Lys $(Xaa)_n$ - $Q^c$

worin jedes n unabhängig eine aus 0, 1, 2 und 3, vorzugsweise 0, gewählte ganze Zahl ist;

jedes Xaa unabhängig irgendeine Aminosäure ist, vorzugsweise eine natürlich vorkommende Aminosäure, vorzugsweise in der L-Konfiguration;

worin jede Aminosäure geschützt oder ungeschützt, vorzugsweise ungeschützt ist, wobei

irgendeine Schutzgruppe für Tyr aus Tetrahydropyranyl, tert-Butyl, Trityl, Bzl, CBZ, 4Br-CBZ und 2,6-Dichlorbenzyl gewählt ist;

irgendeine Schutzgruppe für Asp oder Glu aus Bzl, Cyclohexyl, Cycloheptal, 2,6-Dichlorbenzyl, Methyl und Ethyl gewählt ist;

irgendeine Schutzgruppe für Cys aus MeOBzl, p-Methylbenzyl, Acetamidomethyl, Trityl und Bzl gewählt ist;

irgendeine Schutzgruppe für Thr oder Ser aus Acetyl, Benzoyl, tert-Butyl, Trityl, Tetrahydropyranyl, Bzl, 2,6-Dichlorbenzyl und CBZ gewählt ist;

irgendeine Schutzgruppe für Arg aus Nitro, Tos, CBZ, Adamantyloxycarbonyl und BOC gewählt ist;

irgend eine Schutzgruppe für Lys aus 2-Cl-Z, Tos, CBZ, 1-Amyloxycarbonyl und BOC gewählt ist;

irgendeine Schutzgruppe für Gln oder Asn Xan ist;

irgendeine Schutzgruppe für His aus Tos und Dinitrophenyl gewählt ist;

Qa aus Waserstoff und einer Amino-endständigen Blockierungsgruppe, vorzugsweise Wasserstoff, gewählt ist, wobei

irgendeine Amino-endständige Blockierungsgruppe aus einer Fettsäure mit 2-30 Kohlenstoffatomen, einem freien Amin, Acetamid und einer endständigen Amino-Schutzgruppe gewählt ist, wobei die endständige Amino-Schutzgruppe aus Blockierungsgruppen vom Acyltyp, Blockierungsgruppen vom Typ aromatisches Urethan, Blockierungsgruppen vom Typ aliphatisches Urethan, Blockierungsgruppen vom Cycloalkylurethan-Typ, Blockierungsgruppen vom Thiourethan-Typ, Blockierungsgruppen vom Alkyltyp und Blockierungsgruppen vom Trialkylsilantyp gewählt ist; vorzugsweise eine Fettsäure;

$Q^c$ aus Wasserstoff und einer Carboxy-endständigen Blockierungsgruppe, vorzugsweise Wasserstoff, gewählt ist, wobei

irgendeine Carboxy-endständige Blockierungsgruppe aus einem Fettamin mit 2 bis 30 Kohlenstoffatomen, einer freien Säure, einem Amid und einer endständigen Carboxyl-Schutzgruppe gewählt ist, wobei die endständige Carboxyl-Schutzgruppe aus $-OCH_2$, Estern, Amiden, Hydroziden, $-O-CH_2$-Harzträger und -NH-Harzträger gewählt ist; vorzugsweise ein Fettamin; und

b. eine sichere und wirksame Menge eines ein erweichendes Mittel umfassenden Trägers für die äußerliche Anwendung

umfaßt.

**2.** Zusammensetzung nach Anspruch 1, wobei das Peptid
Arg Ser Arg Lys (SEQ ID NO: 1) ist.

**3.** Zusammensetzung nach mindestens einem der vorangehenden Ansprüche, welche weiterhin eine sichere und wirksame Menge eines Sonnenschutzmittels umfaßt.

**4.** Zusammensetzung nach mindestens einem der vorangehenden Ansprüche, welche weiterhin eine sichere und wirksame Menge eines Retinoids umfaßt.

**5.** Zusammensetzung nach mindestens einem der vorangehenden Ansprüche, welche weiterhin eine sichere und wirksame Menge eines Chelators umfaßt.

**6.** Zusammensetzung nach mindestens einem der vorangehenden Ansprüche, welche weiterhin eine sichere und wirksame Menge eines entzündungshemmenden Mittels umfaßt.

**7.** Zusammensetzung nach einem der Ansprüche 1-2, welche weiterhin eine sichere und wirksame Menge eines Sonnenschutzmittels und eine sichere und wirksame Menge eines Retinodis umfaßt.

**8.** Zusammensetzung nach mindestens einem der Ansprüche 1-2, welche weiterhin eine sichere und wirksame Menge eines Sonnenschutzmittels und eine sichere und wirksame Menge eines Chelators umfaßt.

**9.** Zusammensetzung nach einem der Ansprüche 1-2, welche weiterhin eine sichere und wirksame Menge eines Sonnenschutzmittels und eine sichere und wirksame Menge eines entzündungshemmenden

Mittels umfaßt.

**10.** Zusammensetzung nach mindestens einem der vorangehenden Ansprüche für die äußerliche Anwendung bei der Regulierung von Hautfalten in der Haut von Säugern.

**11.** Verwendung

a. eines Peptids, vorzugsweise 0,001% bis 20%, bestehend aus der Aminosäuresequenz

$Q^a$ - $(Xaa)_n$ Arg Ser Arg Lys $(Xaa)_n$ - $Q^c$

worin jedes n unabhängig eine aus 0, 1, 2 und 3, vorzugsweise 0, gewählte ganze Zahl ist;

jedes Xaa unabhängig irgendeine Aminosäure ist, vorzugsweise eine natürlich vorkommende Aminosäure, vorzugsweise in der L-Konfiguration;

worin jede Aminosäure geschützt oder ungeschützt, vorzugsweise ungeschützt ist, wobei

irgendeine Schutzgruppe für Tyr aus Tetrahydropyranyl, tert-Butyl, Trityl, Bzl, CBZ, 4Br-CBZ und 2,6-Dichlorbenzyl gewählt ist;

irgendeine Schutzgruppe für Asp oder Glu aus Bzl, Cycloheryl, Cycloheptal, 2,6-Dichlorbenzyl, Methyl und Ethyl gewählt ist;

irgendeine Schutzgruppe für Cys aus MeOBzl, p-Methylbenzyl, Acetamidomethyl, Trityl und Bzl gewählt ist;

irgendeine Schutzgruppe für Thr oder Ser aus Acetyl, Benzoyl, tert-Butyl, Trityl, Tetrahydropyranyl, Bzl, 2,6-Dichlorbenzyl und CBZ gewählt ist;

irgendeine Schutzgruppe für Arg aus Nitro, Tos, CBZ, Adamantyloxycarbonyl und BOC gewählt ist;

irgend eine Schutzgruppe für Lys aus 2-Cl-Z, Tos, CBZ, 1-Amyloxycarbonyl und BOC gewählt ist;

irgendeine Schutzgruppe für Gln oder Asn Xan ist;

irgendeine Schutzgruppe für His aus Tos und Dinitrophenyl gewählt ist;

$Q^a$ aus Waserstoff und einer Amino-endständigen Blockierungsgruppe, vorzugsweise Wasserstoff, gewählt ist, wobei

irgendeine Amino-endständige Blockierungsgruppe aus einer Fettsäure mit 2-30 Kohlenstoffatomen, einem freien Amin, Acetamid und einer endständigen Amino-Schutzgruppe gewählt ist, wobei die endständige Amino-Schutzgruppe aus Blockierungsgruppen vom Acyl-typ, Blockierungsgruppen vom Typ aromatisches Urethan, Blockierungsgruppen vom Typ aliphatisches Urethan, Blockierungsgruppen vom Cycloalkylurethan-Typ, Blockierungsgruppen vom Thiourethan-Typ, Blockierungsgruppen vom Alkyltyp und Blockierungsgruppen vom Trialkylsilantyp gewählt ist; vorzugsweise eine Fettsäure;

$Q^c$ aus Wasserstoff und einer Carboxy-endständigen Blockierungsgruppe, vorzugsweise Wasserstoff, gewählt ist, wobei

irgendeine Carboxy-endständige Blockierungsgruppe aus einem Fettamin mit 2 bis 30 Kohlenstoffatomen, einer freien Säure, einem Amid und einer endständigen Carboxyl-Schutzgruppe gewählt ist, wobei die endständige Carboxyl-Schutzgruppe aus $-OCH_2$, Estern, Amiden, Hydroziden, $-O-CH_2$-Harzträger und -NH-Harzträger gewählt ist; vorzugsweise ein Fettamin; und

b. einer sicheren und wirksamen Menge eines ein erweichendes Mittel enthaltenden Trägers für die äußere Anwendung

bei der Herstellung einer Zusammensetzung für die äußere Anwendung zur Regulierung von Falten in der Haut von Säugern.

## Revendications

**1.** Composition pour application locale pour réguler les rides sur la peau des mammifères, caractérisée en ce qu'elle comprend:

a. un peptide, de préférence de 0,001% à 20%, constitué de la séquence d'acides aminés

$Q^a$ - $(Xaa)_n$ Arg Ser Arg Lys $(Xaa)_n$ - $Q^c$

dans laquelle chaque n est indépendamment un nombre entier choisi parmi 0, 1, 2 et 3, de préférence 0;

chaque Xaa est indépendamment tout acide aminé, de préférence un acide aminé naturel, de préférence en configuration L;

chaque acide aminé est protégé ou non protégé, de préférence non protégé, dans lequel

le groupe protecteur éventuel de Tyr est choisi parmi les groupes tétrahydropyranyle, t-butyle,

trityle, Bzl, CBZ, 4Br-CBZ et 2,6-dichlorobenzyle;

le groupe protecteur éventuel de Asp ou de Glu est choisi parmi les groupes Bzl, cyclohexyle, cycloheptal, 2,6-dichlorobenzyle, méthyle et éthyle;

le groupe protecteur éventuel de Cys est choisi parmi les groupes MeOBzl, p-méthylbenzyle, acétamidométhyle, trityle et Bzl;

le groupe protecteur éventuel de Thr ou de Ser est choisi parmi les groupes acétyle, benzoyle, t-butyle, trityle, tétrahydropyranyle, Bzl, 2,6-dichlorobenzyle et CBZ;

le groupe protecteur éventuel de Arg est choisi parmi les groupes nitro, Tos, CBZ, adamantyloxycarbonyle et BOC;

le groupe protecteur éventuel de Lys est choisi parmi les groupes 2-Cl-Z, Tos, CBZ, l-amyloxycarbonyle et BOC;

le groupe protecteur éventuel de Gln ou de Asn est Xan;

le groupe protecteur éventuel de His est choisi parmi les groupes Tos et dinitrophényle;

$Q^a$ est choisi parmi un atome d'hydrogène et un groupe bloquant l'extrémité amino, de préférence un atome d'hydrogène, dans lequel

le groupe bloquant l'extrémité amino éventuel est choisi parmi un acide gras de 2 à 30 atomes de carbone, une amine libre, un acétamide et un groupe protecteur d'amino terminal dans lequel le groupe protecteur d'amino terminal est choisi parmi les groupes bloquants de type acyle, les groupes bloquants de type uréthane aromatique, les groupes bloquants de type uréthane aliphatique, les groupes bloquants de type uréthane cycloalkylique, les groupes bloquants de type thiouréthane, les groupes bloquants de type alkyle et les groupes bloquants trialkylsilane; de préférence un acide gras;

$Q^c$ est choisi parmi un atome d'hydrogène et un groupe bloquant l'extrémité carboxy, de préférence un atome d'hydrogène, dans lequel

le groupe bloquant l'extrémité carboxy éventuel est choisi parmi une amine grasse de 2 à 30 atomes de carbone, un acide libre, un amide et un groupe protecteur de carboxyle terminal dans lequel le groupe protecteur de carboxyle terminal est choisi parmi $-OCH_2$, les esters, les amides, les hydrazides, un support de résine portant $-O-CH_2-$ et un support de résine portant -NH-; de préférence une amine grasse; et

b. une quantité sans danger et efficace d'un véhicule à usage local comprenant un émollient.

**2.** Composition selon la revendication 1, dans laquelle le peptide est
Arg Ser Arg Lys (SEQ ID n° 1)

**3.** Composition selon l'une quelconque des revendications précédentes, qui comprend aussi une quantité sans danger et efficace d'un agent anti-solaire.

**4.** Composition selon l'une quelconque des revendications précédentes, qui comprend aussi une quantité sans danger et efficace d'un rétinoïde.

**5.** Composition selon l'une quelconque des revendications précédentes, qui comprend aussi une quantité sans danger et efficace d'un chélateur.

**6.** Composition selon l'une quelconque des revendications précédentes, qui comprend aussi une quantité sans danger et efficace d'un agent anti-inflammatoire.

**7.** Composition selon l'une quelconque des revendications 1-2, qui comprend aussi une quantité sans danger et efficace d'un agent anti-solaire et une quantité sans danger et efficace d'un rétinoïde.

**8.** Composition selon l'une quelconque des revendications 1-2, qui comprend aussi une quantité sans danger et efficace d'un agent anti-solaire et une quantité sans danger et efficace d'un chélateur.

**9.** Composition selon l'une quelconque des revendications 1-2, qui comprend aussi une quantité sans danger et efficace d'un agent anti-solaire et une quantité sans danger et efficace d'un agent anti-inflammatoire.

**10.** Composition selon l'une quelconque des revendications précédentes, à utiliser localement pour réguler les rides de la peau sur la peau des mammifères.

**11.** Utilisation, pour fabriquer une composition à appliquer localement pour réguler les rides de la peau des mammifères,

a. d'un peptide, de préférence de 0,001% à 20%, constitué de la séquence d'acides aminés

$Q^a$ - $(Xaa)_n$ Arg Ser Arg Lys $(Xaa)_n$ - $Q^c$

dans laquelle chaque n est indépendamment un nombre entier choisi parmi 0, 1, 2 et 3, de préférence 0;

chaque Xaa est indépendamment tout acide aminé, de préférence un acide aminé naturel, de préférence en configuration L;

chaque acide aminé est protégé ou non protégé, de préférence non protégé, dans lequel

le groupe protecteur éventuel de Tyr est choisi parmi les groupes tétrahydropyranyle, t-butyle, trityle, Bzl, CBZ, 4Br-CBZ et 2,6-dichlorobenzyle;

le groupe protecteur éventuel de Asp ou de Glu est choisi parmi les groupes Bzl, cyclohexyle, cycloheptal, 2,6-dichlorobenzyle, méthyle et éthyle;

le groupe protecteur éventuel de Cys est choisi parmi les groupes MeOBzl, p-méthylbenzyle, acétamidométhyle, trityle et Bzl;

le groupe protecteur éventuel de Thr ou de Ser est choisi parmi les groupes acétyle, benzoyle, t-butyle, trityle, tétrahydropyranyle, Bzl, 2,6-dichlorobenzyle et CBZ;

le groupe protecteur éventuel de Arg est choisi parmi les groupes nitro, Tos, CBZ, adamantyloxycarbonyle et BOC;

le groupe protecteur éventuel de Lys est choisi parmi les groupes 2-Cl-Z, Tos, CBZ, l-amyloxycarbonyle et BOC;

le groupe protecteur éventuel de Gln ou de Asn est Xan;

le groupe protecteur éventuel de His est choisi parmi les groupes Tos et dinitrophényle;

$Q^a$ est choisi parmi un atome d'hydrogène et un groupe bloquant l'extrémité amino, de préférence un atome d'hydrogène, dans lequel

le groupe bloquant l'extrémité amino éventuel est choisi parmi un acide gras de 2 à 30 atomes de carbone, une amine libre, un acétamide et un groupe protecteur d'amino terminal dans lequel le groupe protecteur d'amino terminal est choisi parmi les groupes bloquants de type acyle, les groupes bloquants de type uréthane aromatique, les groupes bloquants de type uréthane aliphatique, les groupes bloquants de type uréthane cycloalkylique, les groupes bloquants de type thiouréthane, les groupes bloquants de type alkyle et les groupes bloquants trialkylsilane; de préférence un acide gras;

$Q^c$ est choisi parmi un atome d'hydrogène et un groupe bloquant l'extrémité carboxy, de préférence un atome d'hydrogène, dans lequel

le groupe bloquant l'extrémité carboxy éventuel est choisi parmi une amine grasse de 2 à 30 atomes de carbone, un acide libre, un amide et un groupe protecteur de carboxyle terminal dans lequel le groupe protecteur de carboxyle terminal est choisi parmi $-OCH_2$, les esters, les amides, les hydrazides, un support de résine portant $-O-CH_2-$ et un support de résine portant -NH-; de préférence une amine grasse; et

b. d'une quantité sans danger et efficace d'un véhicule à usage local comprenant un émollient.

## SEQUENCE LISTING

(1) GENERAL INFORMATION:

(i) APPLICANT: FULMER, ANDREW WAYNE

(ii) TITLE OF INVENTION: COMPOSITIONS FOR REGULATING SKIN WRINKLES COMPRISING AN ARG-SER-ARG-LYS BASED PEPTIDE

(iii) NUMBER OF SEQUENCES: 36

(iv) CORRESPONDENCE ADDRESS:
(A) ADDRESSEE: The Procter & Gamble Company
(B) STREET: 11810 East Miami River Road
(C) CITY: Cincinnati
(D) STATE: Ohio
(E) COUNTRY: U.S.A.
(F) ZIP: 45239-8707

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25

(vi) CURRENT APPLICATION DATA:
(A) APPLICATION NUMBER:
(B) FILING DATE:
(C) CLASSIFICATION:

(viii) ATTORNEY/AGENT INFORMATION:
(A) NAME: Corstanje, Brahm J.
(B) REGISTRATION NUMBER: P-34,804

(ix) TELECOMMUNICATION INFORMATION:
(A) TELEPHONE: 513-245-2858
(B) TELEFAX: 513-741-3012

(2) INFORMATION FOR SEQ ID NO:1:

(i) SEQUENCE CHARACTERISTICS:
(A) LENGTH: 4 amino acids
(B) TYPE: amino acid
(D) TOPOLOGY: linear

(ii) MOLECULE TYPE: peptide

(xi) SEQUENCE DESCRIPTION: SEQ ID NO:1:

```
Arg Ser Arg Lys
1
```

(2) INFORMATION FOR SEQ ID NO:2:

(i) SEQUENCE CHARACTERISTICS:
(A) LENGTH: 6 amino acids
(B) TYPE: amino acid
(D) TOPOLOGY: linear

(ii) MOLECULE TYPE: peptide

(xi) SEQUENCE DESCRIPTION: SEQ ID NO:2:

```
Tyr Arg Ser Arg Lys Tyr
1               5
```

(2) INFORMATION FOR SEQ ID NO:3:

(i) SEQUENCE CHARACTERISTICS:
(A) LENGTH: 5 amino acids
(B) TYPE: amino acid
(D) TOPOLOGY: linear

(ii) MOLECULE TYPE: peptide

(xi) SEQUENCE DESCRIPTION: SEQ ID NO:3:

Tyr Arg Ser Arg Lys
1 5

(2) INFORMATION FOR SEQ ID NO:4:

(i) SEQUENCE CHARACTERISTICS:
(A) LENGTH: 5 amino acids
(B) TYPE: amino acid
(D) TOPOLOGY: linear

(ii) MOLECULE TYPE: peptide

(xi) SEQUENCE DESCRIPTION: SEQ ID NO:4:

Arg Ser Arg Lys Tyr
1 5

(2) INFORMATION FOR SEQ ID NO:5:

(i) SEQUENCE CHARACTERISTICS:
(A) LENGTH: 8 amino acids
(B) TYPE: amino acid
(D) TOPOLOGY: linear

(ii) MOLECULE TYPE: peptide

(xi) SEQUENCE DESCRIPTION: SEQ ID NO:5:

Thr Tyr Arg Ser Arg Lys Tyr Ser
1 5

(2) INFORMATION FOR SEQ ID NO:6:

(i) SEQUENCE CHARACTERISTICS:
(A) LENGTH: 8 amino acids
(B) TYPE: amino acid
(D) TOPOLOGY: linear

(ii) MOLECULE TYPE: peptide

(xi) SEQUENCE DESCRIPTION: SEQ ID NO:6:

Ser Tyr Arg Ser Arg Lys Tyr Thr
1 5

(2) INFORMATION FOR SEQ ID NO:7:

(i) SEQUENCE CHARACTERISTICS:
(A) LENGTH: 8 amino acids
(B) TYPE: amino acid
(D) TOPOLOGY: linear

(ii) MOLECULE TYPE: peptide

(xi) SEQUENCE DESCRIPTION: SEQ ID NO:7:

Ser Tyr Arg Ser Arg Lys Tyr Ser
1 5

(2) INFORMATION FOR SEQ ID NO:8:

(i) SEQUENCE CHARACTERISTICS:
(A) LENGTH: 8 amino acids
(B) TYPE: amino acid
(D) TOPOLOGY: linear

(ii) MOLECULE TYPE: peptide

(xi) SEQUENCE DESCRIPTION: SEQ ID NO:8:

Thr Tyr Arg Ser Arg Lys Tyr Thr
1 5

(2) INFORMATION FOR SEQ ID NO:9:

(i) SEQUENCE CHARACTERISTICS:
(A) LENGTH: 6 amino acids
(B) TYPE: amino acid
(D) TOPOLOGY: linear

(ii) MOLECULE TYPE: peptide

(xi) SEQUENCE DESCRIPTION: SEQ ID NO:9:

Arg Ser Arg Lys Tyr Thr
1 5

(2) INFORMATION FOR SEQ ID NO:10:

(i) SEQUENCE CHARACTERISTICS:
(A) LENGTH: 6 amino acids
(B) TYPE: amino acid
(D) TOPOLOGY: linear

(ii) MOLECULE TYPE: peptide

(xi) SEQUENCE DESCRIPTION: SEQ ID NO:10:

Thr Tyr Arg Ser Arg Lys
1 5

(2) INFORMATION FOR SEQ ID NO:11:

(i) SEQUENCE CHARACTERISTICS:
(A) LENGTH: 6 amino acids
(B) TYPE: amino acid
(D) TOPOLOGY: linear

(ii) MOLECULE TYPE: peptide

(xi) SEQUENCE DESCRIPTION: SEQ ID NO:11:

Arg Ser Arg Lys Tyr Ser
1 5

(2) INFORMATION FOR SEQ ID NO:12:

(i) SEQUENCE CHARACTERISTICS:
(A) LENGTH: 6 amino acids
(B) TYPE: amino acid
(D) TOPOLOGY: linear

(ii) MOLECULE TYPE: peptide

(xi) SEQUENCE DESCRIPTION: SEQ ID NO:12:

Ser Tyr Arg Ser Arg Lys
1 5

(2) INFORMATION FOR SEQ ID NO:13:

(i) SEQUENCE CHARACTERISTICS:
(A) LENGTH: 7 amino acids
(B) TYPE: amino acid
(D) TOPOLOGY: linear

(ii) MOLECULE TYPE: peptide

(xi) SEQUENCE DESCRIPTION: SEQ ID NO:13:

Tyr Arg Ser Arg Lys Tyr Thr
1 5

(2) INFORMATION FOR SEQ ID NO:14:

(i) SEQUENCE CHARACTERISTICS:
(A) LENGTH: 7 amino acids
(B) TYPE: amino acid
(D) TOPOLOGY: linear

(ii) MOLECULE TYPE: peptide

(xi) SEQUENCE DESCRIPTION: SEQ ID NO:14:

Thr Tyr Arg Ser Arg Lys Tyr
1 5

(2) INFORMATION FOR SEQ ID NO:15:

(i) SEQUENCE CHARACTERISTICS:
(A) LENGTH: 7 amino acids
(B) TYPE: amino acid
(D) TOPOLOGY: linear

(ii) MOLECULE TYPE: peptide

(xi) SEQUENCE DESCRIPTION: SEQ ID NO:15:

Tyr Arg Ser Arg Lys Tyr Ser
1 5

(2) INFORMATION FOR SEQ ID NO:16:

(i) SEQUENCE CHARACTERISTICS:
(A) LENGTH: 7 amino acids
(B) TYPE: amino acid
(D) TOPOLOGY: linear

(ii) MOLECULE TYPE: peptide

(xi) SEQUENCE DESCRIPTION: SEQ ID NO:16:

Ser Tyr Arg Ser Arg Lys Tyr
1 5

(2) INFORMATION FOR SEQ ID NO:17:

(i) SEQUENCE CHARACTERISTICS:
(A) LENGTH: 10 amino acids
(B) TYPE: amino acid
(D) TOPOLOGY: linear

(ii) MOLECULE TYPE: peptide

(xi) SEQUENCE DESCRIPTION: SEQ ID NO:17:

Asn Thr Tyr Arg Ser Arg Lys Tyr Ser Ser
1 5 10

(2) INFORMATION FOR SEQ ID NO:18:

(i) SEQUENCE CHARACTERISTICS:
(A) LENGTH: 10 amino acids
(B) TYPE: amino acid
(D) TOPOLOGY: linear

(ii) MOLECULE TYPE: peptide

(xi) SEQUENCE DESCRIPTION: SEQ ID NO:18:

Asn Ser Tyr Arg Ser Arg Lys Tyr Thr Ser
1 5 10

(2) INFORMATION FOR SEQ ID NO:19:

(i) SEQUENCE CHARACTERISTICS:
(A) LENGTH: 10 amino acids
(B) TYPE: amino acid
(D) TOPOLOGY: linear

(ii) MOLECULE TYPE: peptide

(xi) SEQUENCE DESCRIPTION: SEQ ID NO:19:

Asn Ser Tyr Arg Ser Arg Lys Tyr Ser Ser
1 5 10

(2) INFORMATION FOR SEQ ID NO:20:

(i) SEQUENCE CHARACTERISTICS:
(A) LENGTH: 10 amino acids
(B) TYPE: amino acid
(D) TOPOLOGY: linear

(ii) MOLECULE TYPE: peptide

(xi) SEQUENCE DESCRIPTION: SEQ ID NO:20:

Asn Thr Tyr Arg Ser Arg Lys Tyr Thr Ser
1 5 10

(2) INFORMATION FOR SEQ ID NO:21:

(i) SEQUENCE CHARACTERISTICS:
(A) LENGTH: 7 amino acids
(B) TYPE: amino acid
(D) TOPOLOGY: linear

(ii) MOLECULE TYPE: peptide

(xi) SEQUENCE DESCRIPTION: SEQ ID NO:21:

Arg Ser Arg Lys Tyr Thr Ser
1 5

(2) INFORMATION FOR SEQ ID NO:22:

(i) SEQUENCE CHARACTERISTICS:
(A) LENGTH: 7 amino acids
(B) TYPE: amino acid
(D) TOPOLOGY: linear

(ii) MOLECULE TYPE: peptide

(xi) SEQUENCE DESCRIPTION: SEQ ID NO:22:

Asn Thr Tyr Arg Ser Arg Lys
1 5

(2) INFORMATION FOR SEQ ID NO:23:

(i) SEQUENCE CHARACTERISTICS:
(A) LENGTH: 7 amino acids
(B) TYPE: amino acid
(D) TOPOLOGY: linear

(ii) MOLECULE TYPE: peptide

(xi) SEQUENCE DESCRIPTION: SEQ ID NO:23:

Arg Ser Arg Lys Tyr Ser Ser
1               5

(2) INFORMATION FOR SEQ ID NO:24:

(i) SEQUENCE CHARACTERISTICS:
(A) LENGTH: 7 amino acids
(B) TYPE: amino acid
(D) TOPOLOGY: linear

(ii) MOLECULE TYPE: peptide

(xi) SEQUENCE DESCRIPTION: SEQ ID NO:24:

Asn Ser Tyr Arg Ser Arg Lys
1               5

(2) INFORMATION FOR SEQ ID NO:25:

(i) SEQUENCE CHARACTERISTICS:
(A) LENGTH: 8 amino acids
(B) TYPE: amino acid
(D) TOPOLOGY: linear

(ii) MOLECULE TYPE: peptide

(xi) SEQUENCE DESCRIPTION: SEQ ID NO:25:

Tyr Arg Ser Arg Lys Tyr Thr Ser
1 5

(2) INFORMATION FOR SEQ ID NO:26:

(i) SEQUENCE CHARACTERISTICS:
(A) LENGTH: 8 amino acids
(B) TYPE: amino acid
(D) TOPOLOGY: linear

(ii) MOLECULE TYPE: peptide

(xi) SEQUENCE DESCRIPTION: SEQ ID NO:26:

Asn Thr Tyr Arg Ser Arg Lys Tyr
1 5

(2) INFORMATION FOR SEQ ID NO:27:

(i) SEQUENCE CHARACTERISTICS:
(A) LENGTH: 8 amino acids
(B) TYPE: amino acid
(D) TOPOLOGY: linear

(ii) MOLECULE TYPE: peptide

(xi) SEQUENCE DESCRIPTION: SEQ ID NO:27:

Tyr Arg Ser Arg Lys Tyr Ser Ser
1 5

(2) INFORMATION FOR SEQ ID NO:28:

(i) SEQUENCE CHARACTERISTICS:
(A) LENGTH: 8 amino acids
(B) TYPE: amino acid
(D) TOPOLOGY: linear

(ii) MOLECULE TYPE: peptide

(xi) SEQUENCE DESCRIPTION: SEQ ID NO:28:

Asn Ser Tyr Arg Ser Arg Lys Tyr
1 5

(2) INFORMATION FOR SEQ ID NO:29:

(i) SEQUENCE CHARACTERISTICS:
(A) LENGTH: 9 amino acids
(B) TYPE: amino acid
(D) TOPOLOGY: linear

(ii) MOLECULE TYPE: peptide

(xi) SEQUENCE DESCRIPTION: SEQ ID NO:29:

Thr Tyr Arg Ser Arg Lys Tyr Ser Ser
1 5

(2) INFORMATION FOR SEQ ID NO:30:

(i) SEQUENCE CHARACTERISTICS:
(A) LENGTH: 9 amino acids
(B) TYPE: amino acid
(D) TOPOLOGY: linear

(ii) MOLECULE TYPE: peptide

(xi) SEQUENCE DESCRIPTION: SEQ ID NO:30:

Asn Thr Tyr Arg Ser Arg Lys Tyr Ser
1 5

(2) INFORMATION FOR SEQ ID NO:31:

(i) SEQUENCE CHARACTERISTICS:
(A) LENGTH: 9 amino acids
(B) TYPE: amino acid
(D) TOPOLOGY: linear

(ii) MOLECULE TYPE: peptide

(xi) SEQUENCE DESCRIPTION: SEQ ID NO:31:

Ser Tyr Arg Ser Arg Lys Tyr Thr Ser
1 5

(2) INFORMATION FOR SEQ ID NO:32:

(i) SEQUENCE CHARACTERISTICS:
(A) LENGTH: 9 amino acids
(B) TYPE: amino acid
(D) TOPOLOGY: linear

(ii) MOLECULE TYPE: peptide

(xi) SEQUENCE DESCRIPTION: SEQ ID NO:32:

Asn Ser Tyr Arg Ser Arg Lys Tyr Thr
1 5

(2) INFORMATION FOR SEQ ID NO:33:

(i) SEQUENCE CHARACTERISTICS:
(A) LENGTH: 9 amino acids
(B) TYPE: amino acid
(D) TOPOLOGY: linear

(ii) MOLECULE TYPE: peptide

(xi) SEQUENCE DESCRIPTION: SEQ ID NO:33:

Ser Tyr Arg Ser Arg Lys Tyr Ser Ser
1 5

(2) INFORMATION FOR SEQ ID NO:34:

(i) SEQUENCE CHARACTERISTICS:
(A) LENGTH: 9 amino acids
(B) TYPE: amino acid
(D) TOPOLOGY: linear

(ii) MOLECULE TYPE: peptide

(xi) SEQUENCE DESCRIPTION: SEQ ID NO:34:

Asn Ser Tyr Arg Ser Arg Lys Tyr Ser
1 5

(2) INFORMATION FOR SEQ ID NO:35:

(i) SEQUENCE CHARACTERISTICS:
(A) LENGTH: 9 amino acids
(B) TYPE: amino acid
(D) TOPOLOGY: linear

(ii) MOLECULE TYPE: peptide

(xi) SEQUENCE DESCRIPTION: SEQ ID NO:35:

Thr Tyr Arg Ser Arg Lys Tyr Thr Ser
1 5

(2) INFORMATION FOR SEQ ID NO:36:

(i) SEQUENCE CHARACTERISTICS:
(A) LENGTH: 9 amino acids
(B) TYPE: amino acid
(D) TOPOLOGY: linear

(ii) MOLECULE TYPE: peptide

(xi) SEQUENCE DESCRIPTION: SEQ ID NO:36:

Asn Thr Tyr Arg Ser Arg Lys Tyr Thr
1 5